# EUROPEAN PATENT SPECIFICATION

(11) **EP 3 353 325 B1**
(45) Date of publication and mention of the grant of the patent: **20.03.2024**
(21) Application number: 16778963.5
(22) Date of filing: 23.09.2016
(51) Int. Cl.: C12Q 1/6869

(54) **SINGLE AMPLICON ACTIVATED EXCLUSION PCR**
SINGLE-AMPLIKON-AKTIVIERTE AUSSCHLUSS-PCR
ACP PAR EXCLUSION ACTIVÉE À PRODUIT D'AMPLIFICATION UNIQUE

(30) Priority: 24.09.2015 US 201562232300 P
(43) Date of publication of application: 01.08.2018
(73) Proprietor: AbVitro LLC, Summit, NJ 07901 (US)
(72) Inventor: VIGNEAULT, Francois, Seattle, Washington 98109 (US); BRIGGS, Adrian Wrangham, Seattle, Washington 98109 (US); GOLDFLESS, Stephen J., Seattle, Washington 98109 (US); BELMONT, Brian J., Seattle, Washington 98109 (US)
(74) Representative: Avidity IP
(86) International application number: PCT/US2016/053596
(87) International publication number: WO 2017/053903

(56) References cited:
- EP-A2- 1 061 134
- WO-A1-2014/144495
- ARAH J SPENCER ET AL: "Massively parallel sequencing of single cells by epicPCR links functional genes with phylogenic markers, Supplementary Information", INTERNET CITATION, 22 September 2015 (2015-09-22), pages 1-22, XP002751970, Retrieved from the Internet: URL:http://www.nature.com/ismej/journal/va op/ncurrent/suppinfo/ismej2015124s1.html [retrieved on 2015-12-08] & SPENCER S J ET AL: "Massively parallel sequencing of single cells by epicPCR links functional genes with phylogenetic markers", THE I S M E JOURNAL: MULTIDISCIPLINARY JOURNAL OF MICROBIAL ECOLOGY, NATURE PUBLISHING GROUP, UNITED KINGDOM, vol. 9, no. 1, 22 September 2015 (2015-09-22), pages 1-10, XP002751969, ISSN: 1751-7362, DOI: 10.1038/ISMEJ.2015.124 [retrieved on 2015-09-22]
- S Kwok ET AL: "A guide to the design and use of mismatched and degenerate primers.", Genome Research, vol. 3, no. 4, 1 February 1994 (1994-02-01), pages S39-S47, XP055101314, ISSN: 1088-9051, DOI: 10.1101/gr.3.4.S39

## Description

### BACKGROUND

The use of water-in-oil emulsion droplets to allow single-cell genetic analysis at very high throughput. Single cells are isolated in individual emulsion droplets before attaching droplet-specific barcodes to nucleic acid targets associated with each cell. Once barcodes have been attached, the material is recovered from the emulsion droplets and sequenced on a high throughput sequencing instrument. Using the barcode sequences all sequences derived from nucleic acids in the same original emulsion droplets can be clustered together, allowing analysis of multiple targets at a single cell level. Since emulsion droplets are tens to hundreds of thousands of times smaller than the typical volumes used for reactions in plastic plates, throughput is greatly enhanced allowing thousands to millions of cells to be analyzed in a single experiment.

Attaching droplet-specific barcodes to nucleic acid targets associated with each of many droplet isolated single cells fundamentally requires each droplet to contain many (typically millions) of barcoding DNA molecules of the same sequence. Further the barcoded DNA molecules of a single vessel must be distinct from the barcoded sequence carried by corresponding barcoding molecules in any other droplet. One known method of generating such a situation is to seed the initial emulsion with very low quantities of an oligonucleotide (*e.g*., a barcoding template) that carries a degenerate sequence, to act as a barcode, flanked by common sequences, to allow amplification within the droplet by PCR. In this situation each droplet at the start of the emulsion reaction contains only a single, zero or very few barcoding templates, which are then amplified by PCR to create many (typically millions) of identical copies of themselves within the droplets. If there is some complementary sequence overlap designed between the common sequence portions of the barcoding templates and the target nucleic acids, an "assembly-by-overlap" step can occur during the PCR in which copies of the barcoding template become physically attached to the target nucleic acids, resulting in droplet-specific barcoding of many targets per single cell. Spencer et al. (2015) proposes a massively parallel sequencing of single cells by epicPCR links functional genes with phylogenetic markers.

However, these previously described droplet barcoding by PCR methods are majorly limited by random Poisson dispersal of barcode templates into the droplets. By diluting the initial barcoding templates into the aqueous solution to be entered into emulsion, an accurate average number of barcoding templates per droplet can be reached, as long as the emulsion is monodisperse (droplets are all the same size). However, the exact number of barcoding templates entering each emulsion droplet is controlled by Poisson statistics and cannot be controlled with high precision. Unlike cells or beads, the small size of DNA barcode template molecules prevents coordination of their entry into droplets on a one-by-one basis based on physical exclusion properties. Droplets prepared to contain an average density of barcoding templates per droplet of exactly 1, leads to, for example, only -36% of droplets containing a single barcoding template, while -36% will have zero templates (empty droplets) and -36% will have more than one template (multiple-barcode droplets). Droplets with no barcoding templates are undesirable since cells within those droplets cannot be barcoded and are therefore lost from analysis. Droplets with multiple barcodes are also undesirable since cells within those droplets may be "counted twice" or more times, and downstream analysis may be unable to distinguish two genuinely similar cells barcoded in different droplets from one cell counted twice by two droplet barcodes in the same droplet.

The methods and compositions described herein provide a solution to the limitation of Poisson dispersal of barcoding templates into emulsion droplets.

### SUMMARY

The invention is defined in the appended claims.

Described herein are methods and compositions for nucleic acid amplification. Described herein are methods and compositions for reliable PCR amplification. In some embodiments a single DNA template molecule is amplified in over 90% of droplets in a water-in-oil emulsion, thereby overcoming limitations of Poisson statistics, with particular utility in high throughput single-cell barcoding and DNA sequencing methods.

In one aspect, provided herein is a method of producing amplicons comprising amplifying a template barcoded polynucleotide molecule in each of a plurality of vessels comprising a plurality of template barcoded polynucleotide molecules, wherein the amplicons in each vessel of the plurality of vessels comprise a barcode amplified from a single template barcoded polynucleotide molecule, wherein the plurality of vessels comprises 50 vessels.

In one aspect, provided herein is a method of barcoding target polynucleotides comprising amplifying a template barcoded polynucleotide molecule in each of a plurality of vessels comprising a plurality of template barcoded polynucleotide molecules, wherein a target polynucleotide in each vessel of the plurality of vessels comprises a barcode amplified from a single template barcoded polynucleotide molecule, wherein the plurality of vessels comprises 50 vessels.

In one aspect, provided herein is a method of barcoding target polynucleotides comprising; amplifying a template barcoded polynucleotide in each vessel of a plurality of vessels comprising from 2-1000 template barcoded polynucleotides with a different barcode per vessel, and uniquely barcoding a target polynucleotide in each vessel of the plurality of vessels.

In one aspect, provided herein is a method comprising amplifying a template barcoded polynucleotide with a first primer and a second primer in each vessel, each vessel of the plurality of vessels comprising a plurality of template barcoded polynucleotides with different barcodes; wherein the first primer has an affinity to each of the plurality of template barcoded polynucleotides that is about the same and that is as least 5-fold less than the affinity of the second primer to an extension product of the first primer; uniquely barcoding a target polynucleotide in each vessel of the plurality of vessels.

In one aspect, provided herein is a method of barcoding target polynucleotides comprising amplifying a template barcoded polynucleotide molecule in each vessel of a plurality of vessels comprising a plurality of template barcoded polynucleotide molecules, wherein the amplifying comprises amplifying with a primer pair comprising a first primer and a second primer, wherein the first primer a melting temperature to the template barcoded polynucleotide molecule that is lower than the melting temperature of a second primer to an extension product of the first primer; and uniquely barcoding a target polynucleotide in each vessel of the plurality of vessels.

In one aspect, provided herein is a method of barcoding target polynucleotides comprising performing an amplification reaction in each vessel of a plurality of vessels comprising a plurality of template barcoded polynucleotides, the amplification reaction comprising: a first cycle comprising: a first extension reaction, on a template barcoded polynucleotide of the plurality of template barcoded polynucleotides to form a first extension molecule, wherein the extension efficiency of the first extension reaction is once per 2 cycles, performing a second extension reaction on the first extension molecule to form a second extension molecule wherein the extension efficiency of the second extension reaction is at least about 2-fold higher than the extension efficiency of the first extension reaction; and a plurality of further cycles comprising amplifying the first and second extension molecules; wherein a target polynucleotide is uniquely barcoded in each vessel of the plurality of vessels.

In one aspect, provided herein is a method of barcoding target polynucleotides comprising performing an amplification reaction in each vessel of a plurality of vessels comprising plurality of template barcoded polynucleotides, the amplification reaction comprising a plurality of cycles, a first cycle of the plurality of cycles comprising: a first extension reaction with a first primer, on a template barcoded polynucleotide of the plurality of template barcoded polynucleotides to form a first extension molecule, wherein the extension efficiency of the first extension reaction is from about 0.0005% to 20%; performing a second extension reaction with a second primer comprising 100% complementarity to a region of the first extension molecule that is more than 8 bases to form a second extension molecule; amplifying the first and second extension molecules; and uniquely barcoding a target polynucleotide in each vessel of the plurality of vessels.

In one aspect, provided herein is a method of barcoding target polynucleotides comprising performing a first extension reaction comprising extending a first extension primer comprising an activating sequence annealed to an exclusion sequence of a template barcoded polynucleotide, thereby forming an activated barcoded polynucleotide; performing a second extension reaction comprising extending a second extension primer annealed to the activated template barcoded polynucleotide to form a nonexclusionary template barcoded polynucleotide; and amplifying the activated template barcoded polynucleotide and the nonexclusionary template barcoded polynucleotide, thereby barcoding a target polynucleotide in a vessel of a plurality of vessels.

In some embodiments, the method further comprises forming the plurality of vessels before performing a first extension reaction.

In one aspect, provided herein is a method of barcoding target polynucleotides comprising forming a plurality of vessels from a pool of template barcoded polynucleotides comprising different barcodes, wherein the plurality of vessels comprises an average of 2 or more of the template barcoded polynucleotides per vessel; performing an amplification reaction in each vessel of the plurality of vessels, and uniquely barcoding a target polynucleotide in 80% to 100% of the vessels of the plurality of vessels.

In one aspect, provided herein is a method of barcoding target polynucleotides comprising: forming a plurality of vessels, wherein at least 50% of the vessels of the plurality of vessels comprise a plurality of template barcoded polynucleotide molecules; performing an amplification reaction in each vessel of the plurality of vessels; and uniquely barcoding a target polynucleotide in in 80% to 100% of the vessels of the plurality of vessels.

In some embodiments, the method further comprises sequencing amplicons of the amplifying.

In some embodiments, the first and the second extension reactions are a first PCR cycle.

In some embodiments, the amplifying comprises one or more PCR cycles. In some embodiments, the amplifying comprises a plurality of PCR cycles. In some embodiments, the amplifying comprises at least 20, 30, 40, 50, 60, 70, 80, 90, or 100 PCR cycles. In some embodiments, the amplifying comprises from 20-200, 20-150, 20-100, 40-200, 40-150, 40-100, 60-200, 60-150, 60-100, 80-200, 80-150, or 80-100 PCR cycles.

In some embodiments, the activated barcoded polynucleotide in a vessel is formed at least one PCR cycle before a second activated barcode is formed in the same vessel. In some embodiments, the activated barcoded polynucleotide in a vessel is formed at least 2, 3, 4, 5, 6, 7, 8, 9, or 10 PCR cycles before a second activated barcode is formed in the same vessel. In some embodiments, the activated barcoded polynucleotide in a vessel is formed at most 10 PCR cycles before an activated barcoded polynucleotide is formed in any other vessel. In some embodiments, each activated template barcoded polynucleotide formed in a vessel comprises the same barcode sequence.

In some embodiments, at least 70%, 80%, 90%, 95%, 98%, 99%, or 100% of products of the amplifying in a vessel comprise a same barcode sequence. In some embodiments, less than 10%, 5%, 4%, 3%, 2%, 1%, or 0.1% of products of the amplifying in a vessel comprise a different barcode sequence. In some embodiments, at least 70%, 80%, 90%, 95%, 98%, 99%, or 100% of the barcoded target polynucleotides or amplified products thereof in a vessel comprise the same barcode sequence. In some embodiments, at least 70%, 80%, 90%, 95%, 98%, 99%, or 100% of the vessels of the plurality of vessels comprises an amount of products of the amplifying that have the same barcode that is at least 70%, 80%, 90%, 95%, 98%, 99%, or 100% of a total amount of barcoded target polynucleotides or amplified products.

In some embodiments, a single activated template barcoded polynucleotide is formed in a vessel of the plurality of vessels. In some embodiments, a single activated template barcoded polynucleotide is formed in at least 50%, 60%, 70%, 80%, 90% or 100% of the vessels of the plurality of vessels. In some embodiments, a single activated template barcoded polynucleotide is formed in each vessel of the plurality of vessels. In some embodiments, the plurality of vessels comprises at least 10, 50, 100, 1,000, 10,000, 100,000, 1,000,000, 10,000,000, or more vessels.

In some embodiments, the vessel is a well, an emulsion, or a droplet.

In some embodiments, the extension efficiency of the first extension reaction is less than once per PCR cycle. In some embodiments, the extension efficiency of the first extension reaction is less than once per 2 PCR cycles. In some embodiments, the extension efficiency of the first extension reaction is less than once per 3, 4, 5, 6, 7, 8, 9, 10, 11, 12, 13, 14, or 15 PCR cycles. In some embodiments, the extension efficiency of the first extension reaction is about once per 3, 4, 5, 6, 7, 8, 9, 10, 11, 12, 13, 14, or 15 PCR cycles. In some embodiments, the extension efficiency of the first extension reaction is less than 80%. In some embodiments, the extension efficiency of the first extension reaction is less than 70%, 60%, 50%, 10%, 1%., 0.1%, or 0.01%. In some embodiments, the extension efficiency of the first extension reaction is from about 0.0005% to 20%. In some embodiments, the extension efficiency of the first extension reaction is from about 0.005% to 5%. In some embodiments, the extension efficiency of the first extension reaction is from about 0.05% to 0.5%. In some embodiments, the extension efficiency of the second extension reaction is at least 80%, 90%, or 100%. In some embodiments, the extension efficiency of the first extension reaction is at least about 2-fold, 5-fold, 10-fold, 25-fold, 50-fold, 100-fold, or 1000-fold lower than the extension efficiency of the second extension reaction. In some embodiments, the extension efficiency of the first extension reaction is at least about 2-fold, 5-fold, 10-fold, 25-fold, 50-fold, 100-fold, or 1000-fold lower than the extension efficiency of an extension reaction of the amplifying. In some embodiments, the amplifying comprises a third extension reaction comprising extending a first extension primer annealed to the nonexclusionary template barcoded polynucleotide. In some embodiments, the extension efficiency of the third extension reaction is at least 80%, 90%, or 100%. In some embodiments, the extension efficiency of the first extension reaction is at least about 2-fold, 5-fold, 10-fold, 25-fold, 50-fold, 100-fold, or 1000-fold lower than the extension efficiency of the third extension reaction. In some embodiments, the extension efficiency of the second extension reaction is about the extension efficiency of an extension reaction of the amplifying. In some embodiments, the extension efficiency of the second extension reaction is about the extension efficiency of the third extension reaction.

In some embodiments, the plurality of vessels comprises an average of at least one template barcoded polynucleotide molecule per vessel. In some embodiments, the plurality of vessels comprises an average of from 1-2000, 1-1000, 1-500, 1-250, 1-100, 1-50, 1-25, 1-10, 1-6, 2-2000, 2-1000, 2-500, 2-250, 2-100, 2-50, 2-25, 2-10, 2-6, 3-2000, 3-1000, 3-500, 3-250, 3-100, 3-50, 3-25, 3-10, or 3-6 template barcoded polynucleotide molecules per vessel. In some embodiments, the plurality of vessels comprises an average of at least 3, 4, 5, 10, 20, 30, 40, 50, 60, 70, 80, 90, 100, 150, 200, 250, 300, 400, 500, 750, or 1000 template barcoded polynucleotide molecules per vessel. In some embodiments, at least 50%, 60%, 70%, 80%, 90% or 100% of the vessels of the plurality of vessels comprise at least one template barcoded polynucleotide molecule. In some embodiments, at least 50%, 60%, 70%, 80%, 90% or 100% of the vessels of the plurality of vessels comprise from 1-2000, 1-1000, 1-500, 1-250, 1-100, 1-50, 1-25, 1-10, or 1-6 template barcoded polynucleotide molecules. In some embodiments, at least 50%, 60%, 70%, 80%, or 90% of the vessels of the plurality of vessels comprise a plurality of template barcoded polynucleotide molecules. In some embodiments, at least 50%, 60%, 70%, 80%, 90% or 100% of the vessels of the plurality of vessels comprise from 2-2000, 2-1000, 2-500, 2-250, 2-100, 2-50, 2-25, 2-10, or 2-6 template barcoded polynucleotide molecules. In some embodiments, each vessel of the plurality of vessels comprises at least 3, 4, 5, 10, 20, 30, 40, 50, 60, 70, 80, 90, 100, 150, 200, 250, 300, 400, 500, 750, or 1000 template barcoded polynucleotide molecules. In some embodiments, at least 50%, 60%, 70%, 80%, 90% or 100% of the vessels of the plurality of vessels comprise from 3-2000, 3-1000, 3-500, 3-250, 3-100, 3-50, 3-25, 3-10, or 3-6 template barcoded polynucleotide molecules.

In some embodiments, a plurality of template barcode polynucleotides is distributed within the plurality of vessels in a Poisson distribution. In some embodiments, a plurality of the first extension primer are distributed within the plurality of vessels in a Poisson distribution.

In some embodiments, the second extension primer in a vessel has 95-100% sequence complementary to the activated template barcoded polynucleotide in the vessel. In some embodiments, the second extension primer in a vessel has 95-100% sequence complementary to the activated template barcoded polynucleotide in different vessel. In some embodiments, the second extension primer in each vessel of the plurality of vessels has 95-100% sequence complementary to the activated template barcoded polynucleotide in different vessel. In some embodiments, the activated template barcoded polynucleotide in a vessel has 95-100% sequence complementary to the second extension primer in the vessel. In some embodiments, the activated template barcoded polynucleotide in a vessel has 95-100% sequence complementary to the second extension primer in a different vessel. In some embodiments, the activated template barcoded polynucleotide in each vessel of the plurality of vessels has 95-100% sequence complementary to the second extension primer in a different vessel. In some embodiments, the activation sequence of a first extension primer in a vessel has less than 95%, 90%, 85%, 80%, 75%, 70%, 65%, 60%, 55%, or 50% sequence complementarity to the exclusion sequence of one or more or each template barcoded polynucleotide in the vessel. In some embodiments, the activation sequence of a first extension primer in a vessel has less than 95%, 90%, 85%, 80%, 75%, 70%, 65%, 60%, 55%, or 50% sequence complementarity to the exclusion sequence of one or more or each template barcoded polynucleotide in a different vessel. In some embodiments, the activation sequence of a first extension primer in each vessel has less than 95%, 90%, 85%, 80%, 75%, 70%, 65%, 60%, 55%, or 50% sequence complementarity to the exclusion sequence of one or more or each template barcoded polynucleotide in a different vessel. In some embodiments, the first extension primer has 100% sequence complementary to less than 15, 14, 13, 12, 11, 10, 9, 8, 7, 6, 5, or 4 bases or less of the activated template barcoded polynucleotide. In some embodiments, the exclusion sequence of the template barcoded polynucleotides in a vessel have 90-100% sequence identity. In some embodiments, the exclusion sequence of a template barcoded polynucleotide in a vessel has 90-100% sequence identity to an exclusion sequence of a template barcoded polynucleotide in a different vessel. In some embodiments, the exclusion sequence of a template barcoded polynucleotide in a vessel has 90-100% sequence identity to an exclusion sequence of a template barcoded polynucleotide in each vessel of the plurality of vessels.

In some embodiments, two or more of template barcoded polynucleotide molecules in one vessel comprise a different barcode sequence. In some embodiments, each template barcoded polynucleotide molecule in one vessel comprises a different vessel barcode. In some embodiments, a barcode of a template barcoded polynucleotide in a vessel is unique to a barcode of a template barcoded polynucleotide in a different vessel. In some embodiments, a barcode of a template barcoded polynucleotide in each vessel is unique to a barcode of a template barcoded polynucleotide in a different vessel.

In some embodiments, the amplifying inhibits amplification of another template barcoded polynucleotide in a same vessel.

In some embodiments, a vessel comprises a cell. In some embodiments, a vessel comprises a single cell. In some embodiments, the target polynucleotide is from the cell.

In some embodiments, at least 70%, 80%, 90%, 95%, 98%, 99%, or 100% of the vessels of the plurality of vessels comprise a barcoded target polynucleotide. In some embodiments, 2 or more target polynucleotides in a vessel are barcoded. In some embodiments, at least 70%, 80%, 90%, 95%, 98%, 99%, or 100% of the vessels of the plurality of vessels comprise 2 or more barcoded target polynucleotides. In some embodiments, the barcoded target polynucleotides in a vessel comprise the same barcode. In some embodiments, the barcoded target polynucleotides in a vessel comprise a different barcode than the barcode of the barcoded target polynucleotides in a different vessel. In some embodiments, the barcoded target polynucleotides in a vessel comprise a different barcode than each of the barcodes of the barcoded target polynucleotides in a different vessel. In some embodiments, each vessel of the plurality of vessels comprises the cell. In some embodiments, each vessel of the plurality of vessels comprises a plurality of primers comprising the first extension primer, the second extension primer, a first amplification primer comprising the activating sequence of the first extension primer, and a second amplification primer complementary to a sequence of the activated barcoded polynucleotide.

In some embodiments, the vessels comprise a polymerase. In some embodiments, the polymerase is a DNA polymerase. In some embodiments, the polymerase is a thermostable polymerase. In some embodiments, a PCR cycle is performed at an annealing temperature of less than 60 °C, 59 °C, 58 °C, 57 °C, 56 °C, 55 °C, 54 °C, 53 °C, 52 °C, 51 °C, 50 °C, 49 °C, 48 °C, 47 °C, 46 °C, or 45 °C. In some embodiments, the first extension primer has melting temperature to a template barcoded polynucleotide that is lower than an annealing temperature of a PCR cycle. In some embodiments, the first extension primer has melting temperature to a template barcoded polynucleotide is lower than an annealing temperature of a PCR cycle by at least 1 °C. In some embodiments, the first extension primer has melting temperature to a template barcoded polynucleotide is lower than an annealing temperature of a PCR cycle by at least 2 °C, 3 °C, 4 °C, 5 °C, 6 °C, 7 °C, 8 °C, 9 °C, 10 °C, 11 °C, 12 °C, 13 °C, 14 °C, or 15 °C.

In some embodiments, the activation sequence comprises a mismatch to the exclusion sequence. In some embodiments, the activation sequence comprises at least 2, 3, 4, 5, 6, 7, 8, 9, or 10 mismatches to the exclusion sequence.

In some embodiments, the target polynucleotide is DNA. In some embodiments, the target nucleic acid is RNA. In some embodiments, the RNA is mRNA.

In some embodiments, the method further comprises an extension primer blocking oligonucleotide. In some embodiments, a reverse transcription reaction comprises reverse transcribing the target polynucleotide (e.g., transcribing RNA into a corresponding sequence of DNA). In some embodiments, the plurality of vessels comprises an extension primer blocking oligonucleotide. In some embodiments, the extension primer blocking oligonucleotide prevents transcription of the target polynucleotide from the extension primer or amplification primer before PCR. In some embodiments, the extension primer blocking oligonucleotide prevents extension of the extension primer or amplification primerbefore PCR.

In some embodiments, the reverse transcription reaction occurs before the amplifying or the amplification reaction. In some embodiments, the reverse transcription reaction is before the barcoding. In some embodiments, the reverse transcription reaction is before the first extension reaction. In some embodiments, the reverse transcription reaction is before the second extension reaction. In some embodiments, the reverse transcription reaction is after the forming of a plurality of vessels.

In some embodiments, the extension primer blocking oligonucleotide has a melting temperature to a template barcoded polynucleotide of the plurality of template barcoded polynucleotides that is higher than the melting temperature of an extension primer or amplification primer to the template barcoded polynucleotide of the plurality of template barcoded polynucleotides. In some embodiments, the extension primer blocking oligonucleotide has a melting temperature to a template barcoded polynucleotide of the plurality of template barcoded polynucleotides that is at least 1 °C, 2 °C, 3 °C, 4 °C, 5 °C, 6 °C, 7 °C, 8 °C, 9 °C, 10 °C, 11 °C, 12 °C, 13 °C, 14 °C, 15 °C, 16 °C, 17 °C, 18 °C, 19 °C, 20 °C, 21 °C, 22 °C, 23 °C, 24 °C, 25 °C, 26 °C, 27 °C, 28 °C, 29 °C, or 30 °C higher than the melting temperature of an extension primer or amplification primer to the template barcoded polynucleotide of the plurality of template barcoded polynucleotides. In some embodiments, the extension primer blocking oligonucleotide has a melting temperature to the plurality of template barcoded polynucleotides that is higher than the melting temperature of an extension primer or amplification primer to the plurality of template barcoded polynucleotides.

In some embodiments, the extension primer blocking oligonucleotide is annealed to the template barcoded polynucleotides during the reverse transcription reaction. In some embodiments, the extension primer blocking oligonucleotide is not annealed to the template barcoded polynucleotides during the amplifying or the amplification reaction, such as PCR.

In some embodiments, the extension primer or amplification primer hybridizes to a region of the template barcoded polynucleotides that hybridizes to the extension primer blocking oligonucleotide. In some embodiments, the region of the template barcoded polynucleotides that hybridizes to the extension primer or amplification primer is shorter than a region of the template barcoded polynucleotide to which the extension primer blocking oligonucleotide hybridizes. In some embodiments, the extension primer or amplification primer hybridizes to a first region of the template barcoded polynucleotides and the extension primer blocking oligonucleotide hybridizes to a second region of the template barcoded polynucleotides, wherein the first region and the second region overlap.

In some embodiments, the extension primer blocking oligonucleotide is longer than the first extension primer. In some embodiments, the extension primer blocking oligonucleotide hybridizes to a region of the plurality of template barcoded polynucleotides comprising the exclusionary sequence. In some embodiments, the region of the plurality of template barcoded polynucleotides comprising the exclusionary sequence to which the extension primer blocking oligonucleotide hybridizes is longer than the exclusionary sequence.

In one aspect, provided herein is a kit, wherein the kit comprises a plurality of template barcoded polynucleotides comprising an exclusionary sequence, a first extension primer comprising an activating sequence with complementarity to an exclusionary sequence, and a second extension primer complementary to the activated barcoded polynucleotide. In some embodiments, the kit further comprises a first amplification primer with complementarity to the exclusion sequence and a second amplification primer complementary to the activated barcoded polynucleotide.

In one aspect, provided herein is a kit comprising a plurality of template barcoded polynucleotides comprising an exclusionary sequence; and a plurality of primers comprising a first extension primer comprising an activating sequence with complementarity to an exclusionary sequence, and a second extension primer complementary to the activated barcoded polynucleotide.

In some embodiments, the kit further comprises a first amplification primer with complementarity to the exclusion sequence and a second amplification primer complementary to the activated barcoded polynucleotide.

In some embodiments, the kit further comprises an extension primer blocking oligonucleotide.

In some embodiments, the extension primer blocking oligonucleotide has a melting temperature to a template barcoded polynucleotide of the plurality of template barcoded polynucleotides that is higher than the melting temperature of the first extension primer to the template barcoded polynucleotide of the plurality of template barcoded polynucleotides.

In some embodiments, the extension primer blocking oligonucleotide has a melting temperature to a template barcoded polynucleotide of the plurality of template barcoded polynucleotides that is at least 1 °C, 2 °C, 3 °C, 4 °C, 5 °C, 6 °C, 7 °C, 8 °C, 9 °C, 10 °C, 11 °C, 12 °C, 13 °C, 14 °C, 15 °C, 16 °C, 17 °C, 18 °C, 19 °C, 20 °C, 21 °C, 22 °C, 23 °C, 24 °C, 25 °C, 26 °C, 27 °C, 28 °C, 29 °C, or 30 °C higher than the melting temperature of the first extension primer to the template barcoded polynucleotide of the plurality of template barcoded polynucleotides.

In some embodiments, the extension primer blocking oligonucleotide has a melting temperature to the plurality of template barcoded polynucleotides that is higher than the melting temperature of the first extension primer to the plurality of template barcoded polynucleotides.

In some embodiments, the extension primer blocking oligonucleotide is longer than the first extension primer.

In some embodiments, the extension primer blocking oligonucleotide hybridizes to a region of the plurality of template barcoded polynucleotides comprising the exclusionary sequence.

In some embodiments, the region of the plurality of template barcoded polynucleotides comprising the exclusionary sequence to which the extension primer blocking oligonucleotide hybridizes is longer than the exclusionary sequence.

In one aspect, provided herein is a composition comprising a plurality of vessels, each vessel of the plurality comprising a plurality of template barcoded polynucleotide molecules and a target polynucleotide comprising a barcode amplified from a single template barcoded polynucleotide molecule of the plurality of template barcoded polynucleotide molecules.

In one aspect, provided herein is a composition, the composition comprising a plurality of vessels, each vessel of the plurality comprising a plurality of template barcoded polynucleotides each with a different barcode, and a target polynucleotide comprising a unique barcode amplified from a template barcoded polynucleotide of the plurality of template barcoded polynucleotides.

In one aspect, provided herein is a composition comprising a plurality of vessels, each vessel of the plurality comprising a plurality of template barcoded polynucleotides, a first primer with an affinity to each template barcoded polynucleotide that is about the same, a second primer with an affinity to an extension product of the first primer that is as least 5-fold more than the affinity of the first primer to each of the template barcoded polynucleotides, and a target polynucleotide comprising a unique barcode amplified from a template barcoded polynucleotide a of the plurality of template barcoded polynucleotides.

In some embodiments, the plurality of vessels comprises at least 50 vessels. In some embodiments, each vessel of the plurality comprises an extension primer blocking oligonucleotide. In some embodiments, each vessel of the plurality comprises a polymerase. In some embodiments, each vessel of the plurality comprises a reverse transcriptase.

### BRIEF DESCRIPTION OF THE DRAWINGS

The novel features described herein are set forth with particularity in the appended claims. A better understanding of the features and advantages of the features described herein will be obtained by reference to the following detailed description that sets forth illustrative examples, in which the principles of the features described herein are utilized, and the accompanying drawings of which:

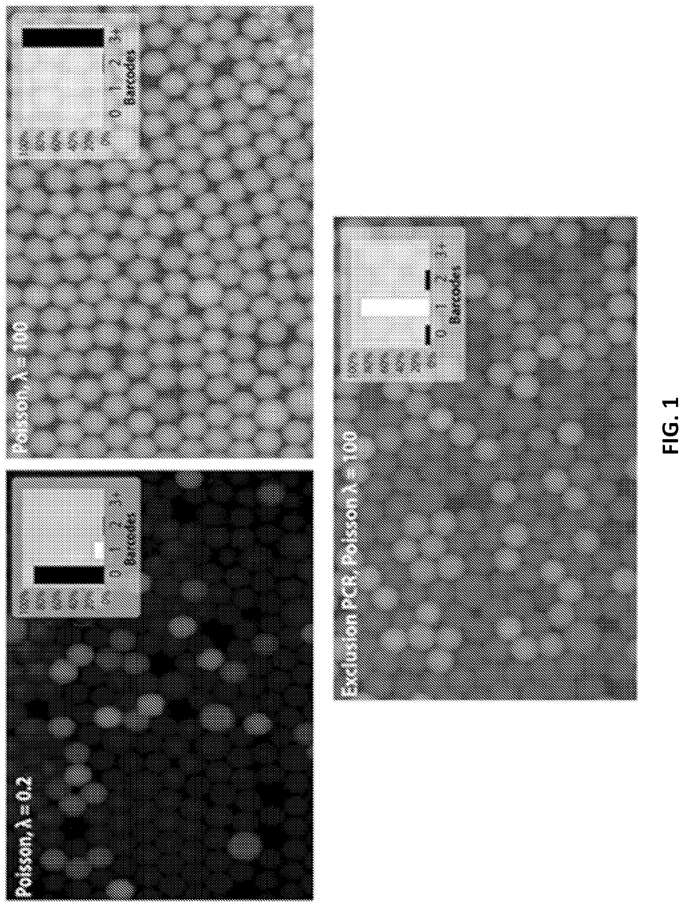

**FIG. 1** depicts an exemplary 3-color PCR-probe assay demonstrating proof-of-principle for SASA-PCR. Single amplicon droplets can be created (right) at much higher frequencies than predicted by Poisson statistics.

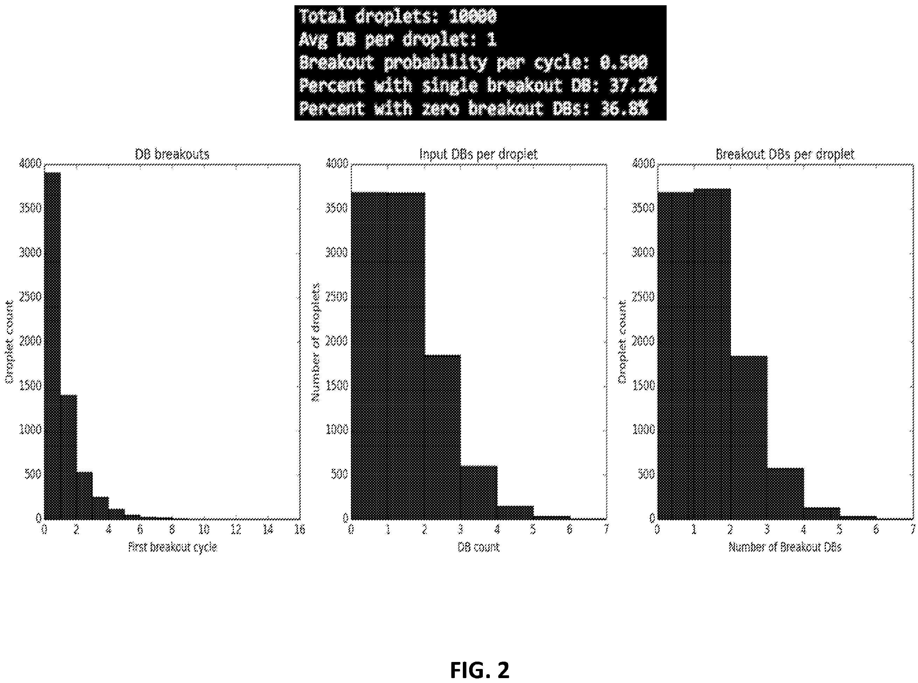

**FIG. 2** depicts an exemplary standard barcoding simulation. Under standard conditions, the Poisson loading of an average of one droplet barcode (DB) template per droplet and high efficiency activation probability (breakout) is expected to yield many droplets (>60%) with either zero or multiple DBs. Breakout DBs are defined as the number of DBs which begin amplification within five PCR amplification cycles of the first DB beginning amplification and includes the initial activated DB. Histograms of the cycle of the first DB breakout, total DB templates per droplet distribution and number of breakout DBs per droplet are shown.

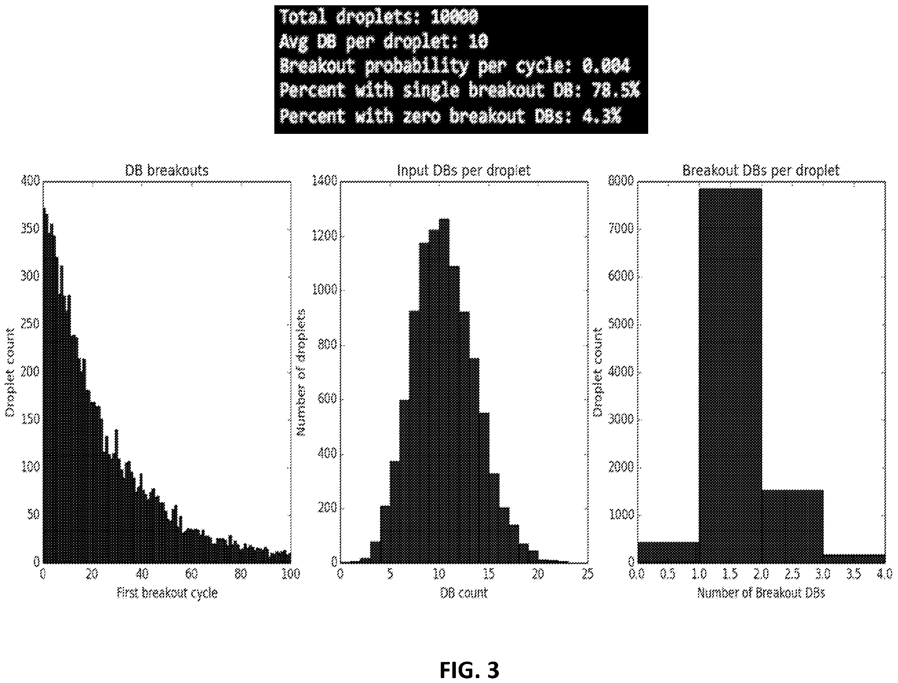

**FIG. 3** depicts an exemplary amplification barcoding simulation. Under improved conditions, the Poisson loading of an average of 10 droplet barcode (DB) templates per droplet and low efficiency activation probability (breakout) is expected to yield a high proportion (78.5%) of single DB droplets and few zero or multiple DB droplets. Breakout DBs are defined as the number of DBs which begin amplification within five PCR amplification cycles of the first DB beginning amplification and includes the initial activated DB. Histograms of the cycle of the first DB breakout, total DB templates per droplet distribution and number of breakout DBs per droplet are shown.

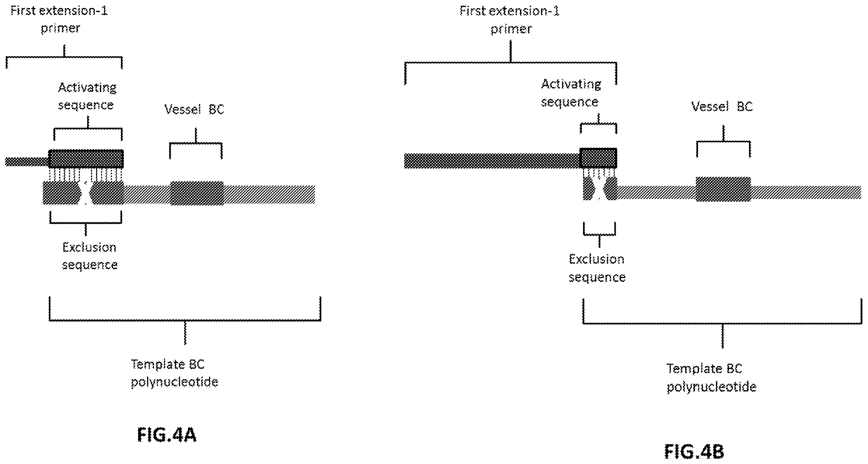

**FIG. 4A** depicts an exemplary design of a primer for use in the described methods.
**FIG. 4B** depicts an exemplary design of a primer for use in the described methods.

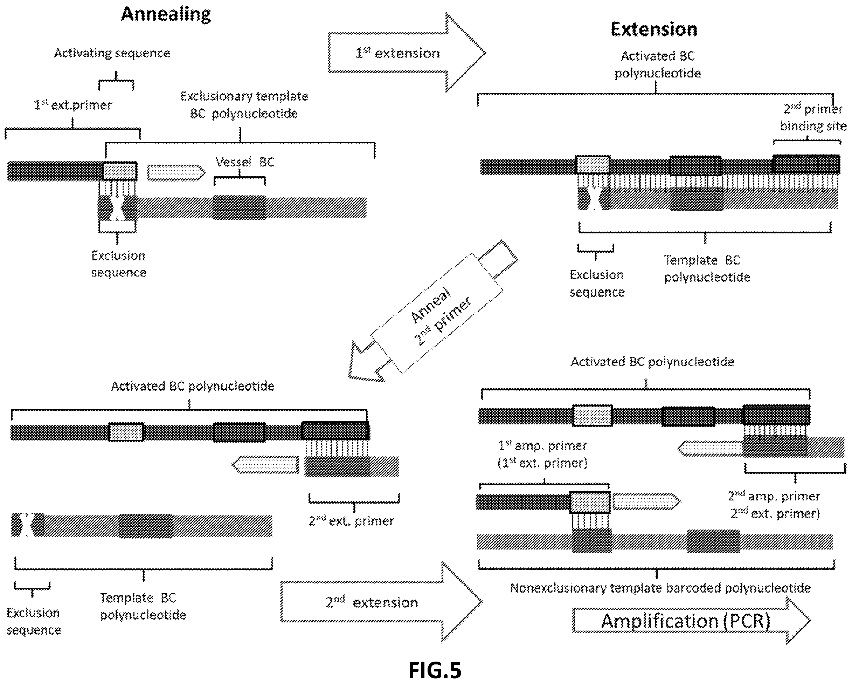

**FIG. 5** depicts an exemplary schematic of the described methods.
**FIG. 6** depicts an exemplary schematic of a method for preventing extension of an extension primer during reverse transcription (RT) before PCR for RT-PCR applications such as emulsion barcoding.
**FIG. 6A** depicts a schematic of an exemplary extension reaction. The extension primer is designed to overlap with the template such that the extension primer is extended at a known low frequency in PCR conditions with PCR polymerase.
**FIG. 6B** depicts a schematic of an exemplary reverse transcription reaction without an extension primer blocking oligonucleotide. Because reverse transcription occurs at a lower temperature than PCR and with a different enzyme, there is a high potential for extension before the first PCR cycle.
**FIG. 6C** depicts a schematic of an exemplary reverse transcription reaction with an extension primer blocking oligonucleotide. The extension primer blocking oligonucleotide is designed to have a higher T_{M} than the T_{M} of the extension primer. Annealing the extension primer blocking oligonucleotide to the template prevents access of the extension primer to the template during reverse transcription, and prevents extension of the extension primer before PCR.
**FIG. 7** depicts an exemplary graph of relative fluorescent units (RFU) vs. number of PCR cycles for the method depicted in **FIG. 6C**. Real-time PCR was carried out to amplify 1× 10⁷ templates in the presence of (1) a low-efficiency extension primer and a PCR polymerase, (2) a PCR polymerase and a reverse transcriptase, or (3) a PCR polymerase, a reverse transcriptase, and an extension primer blocking oligonucleotide pre-annealed to the templates.

### DETAILED DESCRIPTION

Several aspects are described below with reference to example applications for illustration. It should be understood that numerous specific details, relationships, and methods are set forth to provide a full understanding of the features described herein. One having ordinary skill in the relevant art, however, will readily recognize that the features described herein can be practiced without one or more of the specific details or with other methods. The features described herein are not limited by the illustrated ordering of acts or events, as some acts can occur in different orders and/or concurrently with other acts or events. Furthermore, not all illustrated acts or events are required to implement a methodology in accordance with the features described herein.

The terminology used herein is for the purpose of describing particular cases only and is not intended to be limiting. As used herein, the singular forms "a", "an" and "the" are intended to include the plural forms as well, unless the context clearly indicates otherwise. Furthermore, to the extent that the terms "including", "includes", "having", "has", "with", or variants thereof are used in either the detailed description and/or the claims, such terms are intended to be inclusive in a manner similar to the term "comprising".

The term "about" or "approximately" can mean within an acceptable error range for the particular value as determined by one of ordinary skill in the art, which will depend in part on how the value is measured or determined, *i.e*., the limitations of the measurement system. For example, "about" can mean within 1 or more than 1 standard deviation, per the practice in the art. Alternatively, "about" can mean a range of up to 20%, up to 10%, up to 5%, or up to 1% of a given value. Alternatively, particularly with respect to biological systems or processes, the term can mean within an order of magnitude, within 5-fold, and more preferably within 2-fold, of a value. Where particular values are described in the application and claims, unless otherwise stated the term "about" meaning within an acceptable error range for the particular value should be assumed.

Compared to other methods of droplet barcoding PCR, the methods and compositions described herein greatly increases (e.g., >90%) the proportion of all droplets in which one single droplet barcode sequence is successfully amplified in each droplet. Further, compared to other methods of droplet barcoding PCR, the methods and compositions described herein greatly reduce the total number and proportions of empty droplets and droplets containing multiple-barcodes that are amplified. The stochastic activation-exclusion methods and compositions of this disclosure can be used to overcome the limitations of Poisson distribution of barcoded templates in a novel and previously undescribed way.

The experiments of the inventors have demonstrated that primer design according to the description above can, for example, be used create a situation where each template barcoded polynucleotide has about a 0.01% chance per PCR cycle to be extended on by the activating primer. At such a level, a droplet containing 200 template barcoded polynucleotides has about a ~2% chance at each amplification (i.e. PCR) cycle of any of its template barcoded polynucleotides being extended. This leads to the result where the first "activated" barcoding template in a droplet is likely to be activated several PCR cycles before the second activated barcoding template. Once any single activating primer has been successfully extended on a template barcoded polynucleotide, the product of this extension (i.e. the activated template) will contain the sequence of the activating primer that was extended.

Simulations show that 100 standard PCR cycles provide good results. However, a large number of PCR cycles may inhibit and deactivate thermostable polymerases over time under standard PCR cycling conditions. A solution to this issue provided by the described methods and compositions. In some embodiments, a method comprises the use of lower than standard denaturation temperatures in the PCR, which are still sufficient for amplification of the droplet barcodes but milder on the polymerase allowing it to perform for longer periods and more cycles than is common. In some embodiments, a method comprises performing a number of PCR cycles at a lower denaturation temperature than standard saturation temperatures in the PCR. In some embodiments, a method comprises performing a number of PCR cycles at a lower denaturation temperature than standard saturation temperatures in the PCR, wherein the lower denaturation temperature is sufficient for amplification of template barcode polynucleotides. In some embodiments, a method comprises performing a number of PCR cycles at a lower denaturation temperature than standard saturation temperatures in the PCR.

In one aspect, polynucleotides template molecules comprising a barcode sequence can be isolated into individual droplets at a relatively high density of several templates per droplet. In some embodiments, a plurality of droplets can be created or obtained, wherein the number of individual barcode template molecules in each vessel is tightly controlled. In some embodiments, a plurality of droplets can be created or obtained, wherein the number of individual barcode template molecules in each vessel is from 3 per droplet to 1000 per droplet. The methods described herein can comprise performing a first strand extension reaction. In some embodiments, a first extension reaction extends a primer (e.g., a PCR primer) attached to a template barcoded polynucleotide. In some embodiments, the first extension reaction primer has a relatively low affinity to the template barcoded polynucleotide. In some embodiments, the first extension reaction has a rate of initiation of the extension of a primer on a template barcoded polynucleotide that is low. In some embodiments, a primer of the first extension reaction (i.e. an "activating" primer) has a relatively low affinity to a template barcoded polynucleotide, is an efficient PCR primer on a template containing its fully complementary sequence.

In some embodiments, a method comprises a first extension reaction with one or more activating primers, wherein one or more or each of the one or more activating primers has a rate of extension initiation of less than 1% per amplification cycle. In some embodiments, a method comprises a first extension reaction with one or more activating primers, wherein one or more or each of the one or more activating primers has a rate of extension initiation of less than 0.1% per amplification cycle. In some embodiments, a method comprises a first extension reaction with one or more activating primers, wherein one or more or each of the one or more activating primers has a rate of extension initiation of less than 0.01% per amplification cycle. In some embodiments, a method comprises a first extension reaction with one or more activating primers, wherein one or more or each of the one or more activating primers has a rate of extension initiation of less than 0.001% per amplification cycle. For example, a method can comprise a first extension reaction with a primer that has a rate of extension initiation of less than 1% per amplification cycle. For example, a method can comprise a first extension reaction with a primer that has a rate of extension initiation of less than 0.1% per amplification cycle. For example, a method can comprise a first extension reaction with a primer that has a rate of extension initiation of less than 0.01% per amplification cycle. In some embodiments, the first extension reaction can be performed in the presence of two or more primers that each has a rate of extension initiation on a template barcoded polynucleotide that is low. For example, a method can comprise a first extension reaction with two or more primers that each has a rate of extension initiation of less than 1% per amplification cycle. For example, a method can comprise a first extension reaction with two or more primers that each has a rate of extension initiation of less than 0.1% per amplification cycle. For example, a method can comprise a first extension reaction with two or more primers that each has a rate of extension initiation of less than 0.01% per amplification cycle. For example, a method can comprise a first extension reaction with 3 to1000, 3 to 500, 3 to 250, 3 to 100, 3 to 50, 3 to 25, 3 to 10, or 3 to 6 activating primers, wherein one or more or each of the activating primers has a rate of extension initiation of 0.001%-1% per amplification cycle.

In one aspect, provided herein is a method of producing amplicons comprising amplifying a template barcoded polynucleotide molecule in each of a plurality of vessels comprising a plurality of template barcoded polynucleotide molecules, wherein the amplicons in each vessel of the plurality of vessels comprise a barcode amplified from a single template barcoded polynucleotide molecule, wherein the plurality of vessels comprises 50 vessels.

In one aspect, provided herein is a method of barcoding target polynucleotides comprising amplifying a template barcoded polynucleotide molecule in each of a plurality of vessels comprising a plurality of template barcoded polynucleotide molecules, wherein a target polynucleotide in each vessel of the plurality of vessels comprises a barcode amplified from a single template barcoded polynucleotide molecule, wherein the plurality of vessels comprises 50 vessels.

In one aspect, provided herein is a method of barcoding target polynucleotides comprising; amplifying a template barcoded polynucleotide in each vessel of a plurality of vessels comprising from 2-1000 template barcoded polynucleotides with a different barcode per vessel, and uniquely barcoding a target polynucleotide in each vessel of the plurality of vessels.

In one aspect, provided herein is a method comprising amplifying a template barcoded polynucleotide with a first primer and a second primer in each vessel, each vessel of the plurality of vessels comprising a plurality of template barcoded polynucleotides with different barcodes; wherein the first primer has an affinity to each of the plurality of template barcoded polynucleotides that is about the same and that is as least 5-fold less than the affinity of the second primer to an extension product of the first primer; uniquely barcoding a target polynucleotide in each vessel of the plurality of vessels.

In one aspect, provided herein is a method of barcoding target polynucleotides comprising amplifying a template barcoded polynucleotide molecule in each vessel of a plurality of vessels comprising a plurality of template barcoded polynucleotide molecules, wherein the amplifying comprises amplifying with a primer pair comprising a first primer and a second primer, wherein the first primer a melting temperature to the template barcoded polynucleotide molecule that is lower than the melting temperature of a second primer to an extension product of the first primer; and uniquely barcoding a target polynucleotide in each vessel of the plurality of vessels.

In one aspect, provided herein is a method of barcoding target polynucleotides comprising performing an amplification reaction in each vessel of a plurality of vessels comprising a plurality of template barcoded polynucleotides, the amplification reaction comprising: a first cycle comprising: a first extension reaction, on a template barcoded polynucleotide of the plurality of template barcoded polynucleotides to form a first extension molecule, wherein the extension efficiency of the first extension reaction is once per 2 cycles, performing a second extension reaction on the first extension molecule to form a second extension molecule wherein the extension efficiency of the second extension reaction is at least about 2-fold higher than the extension efficiency of the first extension reaction; and a plurality of further cycles comprising amplifying the first and second extension molecules; wherein a target polynucleotide is uniquely barcoded in each vessel of the plurality of vessels.

In one aspect, provided herein is a method of barcoding target polynucleotides comprising performing an amplification reaction in each vessel of a plurality of vessels comprising plurality of template barcoded polynucleotides, the amplification reaction comprising a plurality of cycles, a first cycle of the plurality of cycles comprising: a first extension reaction with a first primer, on a template barcoded polynucleotide of the plurality of template barcoded polynucleotides to form a first extension molecule, wherein the extension efficiency of the first extension reaction is from about 0.0005% to 20%; performing a second extension reaction with a second primer comprising 100% complementarity to a region of the first extension molecule that is more than 8 bases to form a second extension molecule; amplifying the first and second extension molecules; and uniquely barcoding a target polynucleotide in each vessel of the plurality of vessels.

In one aspect, provided herein is a method of barcoding target polynucleotides comprising performing a first extension reaction comprising extending a first extension primer comprising an activating sequence annealed to an exclusion sequence of a template barcoded polynucleotide, thereby forming an activated barcoded polynucleotide; performing a second extension reaction comprising extending a second extension primer annealed to the activated template barcoded polynucleotide to form a nonexclusionary template barcoded polynucleotide; and amplifying the activated template barcoded polynucleotide and the nonexclusionary template barcoded polynucleotide, thereby barcoding a target polynucleotide in a vessel of a plurality of vessels.

In some embodiments, the method further comprises forming the plurality of vessels before performing a first extension reaction.

In one aspect, provided herein is a method of barcoding target polynucleotides comprising forming a plurality of vessels from a pool of template barcoded polynucleotides comprising different barcodes, wherein the plurality of vessels comprises an average of 2 or more of the template barcoded polynucleotides per vessel; performing an amplification reaction in each vessel of the plurality of vessels, and uniquely barcoding a target polynucleotide in 80% to 100% of the vessels of the plurality of vessels.

In one aspect, provided herein is a method of barcoding target polynucleotides comprising: forming a plurality of vessels, wherein at least 50% of the vessels of the plurality of vessels comprise a plurality of template barcoded polynucleotide molecules; performing an amplification reaction in each vessel of the plurality of vessels; and uniquely barcoding a target polynucleotide in in 80% to 100% of the vessels of the plurality of vessels.

In some embodiments, the method further comprises sequencing amplicons of the amplifying. In some embodiments, the first and the second extension reactions are a first PCR cycle. In some embodiments, the amplifying comprises one or more PCR cycles. In some embodiments, the amplifying comprises a plurality of PCR cycles. In some embodiments, the amplifying comprises at least 20, 30, 40, 50, 60, 70, 80, 90, or 100 PCR cycles. In some embodiments, the amplifying comprises from 20-200, 20-150, 20-100, 40-200, 40-150, 40-100, 60-200, 60-150, 60-100, 80-200, 80-150, or 80-100 PCR cycles. In some embodiments, the activated barcoded polynucleotide in a vessel is formed at least one PCR cycle before a second activated barcode is formed in the same vessel. In some embodiments, the activated barcoded polynucleotide in a vessel is formed at least 2, 3, 4, 5, 6, 7, 8, 9, or 10 PCR cycles before a second activated barcode is formed in the same vessel. In some embodiments, the activated barcoded polynucleotide in a vessel is formed at most 10 PCR cycles before an activated barcoded polynucleotide is formed in any other vessel.

In some embodiments, each activated template barcoded polynucleotide formed in a vessel comprises the same barcode sequence. In some embodiments, at least 70%, 80%, 90%, 95%, 98%, 99%, or 100% of products of the amplifying in a vessel comprise a same barcode sequence. In some embodiments, less than 10%, 5%, 4%, 3%, 2%, 1%, or 0.1% of products of the amplifying in a vessel comprise a different barcode sequence. In some embodiments, at least 70%, 80%, 90%, 95%, 98%, 99%, or 100% of the barcoded target polynucleotides or amplified products thereof in a vessel comprise the same barcode sequence. The method of claim of any one of claims 1-24, wherein at least 70%, 80%, 90%, 95%, 98%, 99%, or 100% of the vessels of the plurality of vessels comprises an amount of products of the amplifying that have the same barcode that is at least 70%, 80%, 90%, 95%, 98%, 99%, or 100% of a total amount of barcoded target polynucleotides or amplified products. In some embodiments, a single activated template barcoded polynucleotide is formed in a vessel of the plurality of vessels. In some embodiments, a single activated template barcoded polynucleotide is formed in at least 50%, 60%, 70%, 80%, 90% or 100% of the vessels of the plurality of vessels. In some embodiments, a single activated template barcoded polynucleotide is formed in each vessel of the plurality of vessels.

In some embodiments, the plurality of vessels comprises at least 10, 50, 100, 1,000, 10,000, 100,000, 1,000,000, 10,000,000, or more vessels. In some embodiments, the vessel is a well, an emulsion, or a droplet.

In some embodiments, the extension efficiency of the first extension reaction is less than once per PCR cycle. In some embodiments, the extension efficiency of the first extension reaction is less than once per 2 PCR cycles. In some embodiments, the extension efficiency of the first extension reaction is less than once per 3, 4, 5, 6, 7, 8, 9, 10, 11, 12, 13, 14, or 15 PCR cycles. In some embodiments, the extension efficiency of the first extension reaction is about once per 3, 4, 5, 6, 7, 8, 9, 10, 11, 12, 13, 14, or 15 PCR cycles.

In some embodiments, the extension efficiency of the first extension reaction is less than 80%. In some embodiments, the extension efficiency of the first extension reaction is less than 70%, 60%, 50%, 10%, 1%., 0.1%, or 0.01%. In some embodiments, the extension efficiency of the first extension reaction is from about 0.0005% to 20%. In some embodiments, the extension efficiency of the first extension reaction is from about 0.005% to 5%. In some embodiments, the extension efficiency of the first extension reaction is from about 0.05% to 0.5%. In some embodiments, the extension efficiency of the second extension reaction is at least 80%, 90%, or 100%. In some embodiments, the extension efficiency of the first extension reaction is at least about 2-fold, 5-fold, 10-fold, 25-fold, 50-fold, 100-fold, or 1000-fold lower than the extension efficiency of the second extension reaction. In some embodiments, the extension efficiency of the first extension reaction is at least about 2-fold, 5-fold, 10-fold, 25-fold, 50-fold, 100-fold, or 1000-fold lower than the extension efficiency of an extension reaction of the amplifying. In some embodiments, the amplifying comprises a third extension reaction comprising extending a first extension primer annealed to the nonexclusionary template barcoded polynucleotide. In some embodiments, the extension efficiency of the third extension reaction is at least 80%, 90%, or 100%. In some embodiments, the extension efficiency of the first extension reaction is at least about 2-fold, 5-fold, 10-fold, 25-fold, 50-fold, 100-fold, or 1000-fold lower than the extension efficiency of the third extension reaction. In some embodiments, the extension efficiency of the second extension reaction is about the extension efficiency of an extension reaction of the amplifying. In some embodiments, the extension efficiency of the second extension reaction is about the extension efficiency of the third extension reaction. In some embodiments, the plurality of vessels comprises an average of at least one template barcoded polynucleotide molecule per vessel. In some embodiments, the plurality of vessels comprises an average of from 1-2000, 1-1000, 1-500, 1-250, 1-100, 1-50, 1-25, 1-10, 1-6, 2-2000, 2-1000, 2-500, 2-250, 2-100, 2-50, 2-25, 2-10, 2-6, 3-2000, 3-1000, 3-500, 3-250, 3-100, 3-50, 3-25, 3-10, or 3-6 template barcoded polynucleotide molecules per vessel. In some embodiments, the plurality of vessels comprises an average of at least 3, 4, 5, 10, 20, 30, 40, 50, 60, 70, 80, 90, 100, 150, 200, 250, 300, 400, 500, 750, or 1000 template barcoded polynucleotide molecules per vessel. In some embodiments, at least 50%, 60%, 70%, 80%, 90% or 100% of the vessels of the plurality of vessels comprise at least one template barcoded polynucleotide molecule. In some embodiments, at least 50%, 60%, 70%, 80%, 90% or 100% of the vessels of the plurality of vessels comprise from 1-2000, 1-1000, 1-500, 1-250, 1-100, 1-50, 1-25, 1-10, or 1-6 template barcoded polynucleotide molecules. In some embodiments, at least 50%, 60%, 70%, 80%, or 90% of the vessels of the plurality of vessels comprise a plurality of template barcoded polynucleotide molecules. In some embodiments, at least 50%, 60%, 70%, 80%, 90% or 100% of the vessels of the plurality of vessels comprise from 2-2000, 2-1000, 2-500, 2-250, 2-100, 2-50, 2-25, 2-10, or 2-6 template barcoded polynucleotide molecules. In some embodiments, each vessel of the plurality of vessels comprises at least 3, 4, 5, 10, 20, 30, 40, 50, 60, 70, 80, 90, 100, 150, 200, 250, 300, 400, 500, 750, or 1000 template barcoded polynucleotide molecules. In some embodiments, at least 50%, 60%, 70%, 80%, 90% or 100% of the vessels of the plurality of vessels comprise from 3-2000, 3-1000, 3-500, 3-250, 3-100, 3-50, 3-25, 3-10, or 3-6 template barcoded polynucleotide molecules.

In some embodiments, a plurality of template barcode polynucleotides is distributed within the plurality of vessels in a Poisson distribution. In some embodiments, a plurality of the first extension primer is distributed within the plurality of vessels in a Poisson distribution. In some embodiments, the second extension primer in a vessel has 95-100% sequence complementary to the activated template barcoded polynucleotide in the vessel.

In some embodiments, the second extension primer in a vessel has 95-100% sequence complementary to the activated template barcoded polynucleotide in different vessel. In some embodiments, the second extension primer in each vessel of the plurality of vessels has 95-100% sequence complementary to the activated template barcoded polynucleotide in different vessel. In some embodiments, the activated template barcoded polynucleotide in a vessel has 95-100% sequence complementary to the second extension primer in the vessel. In some embodiments, the activated template barcoded polynucleotide in a vessel has 95-100% sequence complementary to the second extension primer in a different vessel. In some embodiments, the activated template barcoded polynucleotide in each vessel of the plurality of vessels has 95-100% sequence complementary to the second extension primer in a different vessel. In some embodiments, the activation sequence of a first extension primer in a vessel has less than 95%, 90%, 85%, 80%, 75%, 70%, 65%, 60%, 55%, or 50% sequence complementarity to the exclusion sequence of one or more or each template barcoded polynucleotide in the vessel. In some embodiments, the activation sequence of a first extension primer in a vessel has less than 95%, 90%, 85%, 80%, 75%, 70%, 65%, 60%, 55%, or 50% sequence complementarity to the exclusion sequence of one or more or each template barcoded polynucleotide in a different vessel. In some embodiments, the activation sequence of a first extension primer in each vessel has less than 95%, 90%, 85%, 80%, 75%, 70%, 65%, 60%, 55%, or 50% sequence complementarity to the exclusion sequence of one or more or each template barcoded polynucleotide in a different vessel. In some embodiments, the first extension primer has 100% sequence complementary to less than 15, 14, 13, 12, 11, 10, 9, 8, 7, 6, 5, or 4 bases or less of the activated template barcoded polynucleotide. The method of claim of any one of claims 1-68, wherein the exclusion sequence of the template barcoded polynucleotides in a vessel have 90-100% sequence identity. The method of claim of any one of claims 1-69, wherein the exclusion sequence of a template barcoded polynucleotide in a vessel has 90-100% sequence identity to an exclusion sequence of a template barcoded polynucleotide in a different vessel. The method of claim of any one of claims 1-70, wherein the exclusion sequence of a template barcoded polynucleotide in a vessel has 90-100% sequence identity to an exclusion sequence of a template barcoded polynucleotide in each vessel of the plurality of vessels. In some embodiments, two or more of template barcoded polynucleotide molecules in one vessel comprise a different barcode sequence.

In some embodiments, each template barcoded polynucleotide molecule in one vessel comprises a different vessel barcode. The method of claim of any one of claims 1-73, wherein a barcode of a template barcoded polynucleotide in a vessel is unique to a barcode of a template barcoded polynucleotide in a different vessel. The method of claim of any one of claims 1-74, wherein a barcode of a template barcoded polynucleotide in each vessel is unique to a barcode of a template barcoded polynucleotide in a different vessel. In some embodiments, the amplifying inhibits amplification of another template barcoded polynucleotide in a same vessel.

In some embodiments, a vessel comprises a cell.

In some embodiments, a vessel comprises a single cell. In some embodiments, the target polynucleotide is from the cell.

In some embodiments, at least 70%, 80%, 90%, 95%, 98%, 99%, or 100% of the vessels of the plurality of vessels comprise a barcoded target polynucleotide. In some embodiments, 2 or more target polynucleotides in a vessel are barcoded. In some embodiments, at least 70%, 80%, 90%, 95%, 98%, 99%, or 100% of the vessels of the plurality of vessels comprise 2 or more barcoded target polynucleotides. The method of claim of any one of claims 1-82, wherein the barcoded target polynucleotides in a vessel comprise the same barcode. The method of claim of any one of claims 1-83, wherein the barcoded target polynucleotides in a vessel comprise a different barcode than the barcode of the barcoded target polynucleotides in a different vessel. The method of claim of any one of claims 1-84, wherein the barcoded target polynucleotides in a vessel comprise a different barcode than each of the barcodes of the barcoded target polynucleotides in a different vessel. In some embodiments, each vessel of the plurality of vessels comprises the cell.

In some embodiments, each vessel of the plurality of vessels comprises a plurality of primers comprising the first extension primer, the second extension primer, a first amplification primer comprising the activating sequence of the first extension primer, and a second amplification primer complementary to a sequence of the activated barcoded polynucleotide.

In some embodiments, the vessels comprise a polymerase. In some embodiments, the polymerase is a DNA polymerase. In some embodiments, the polymerase is a thermostable polymerase.

In some embodiments, a PCR cycle is performed at an annealing temperature of less than 60 °C, 59 °C, 58 °C, 57 °C, 56 °C, 55 °C, 54 °C, 53 °C, 52 °C, 51 °C, 50 °C, 49 °C, 48 °C, 47 °C, 46 °C, or 45 °C. In some embodiments, the first extension primer has melting temperature to a template barcoded polynucleotide that is lower than an annealing temperature of a PCR cycle. In some embodiments, the first extension primer has melting temperature to a template barcoded polynucleotide is lower than an annealing temperature of a PCR cycle by at least 1 °C. In some embodiments, the first extension primer has melting temperature to a template barcoded polynucleotide is lower than an annealing temperature of a PCR cycle by at least 2 °C, 3 °C, 4 °C, 5 °C, 6 °C, 7 °C, 8 °C, 9 °C, 10 °C, 11 °C, 12 °C, 13 °C, 14 °C, or 15 °C. In some embodiments, the activation sequence comprises a mismatch to the exclusion sequence. In some embodiments, the activation sequence comprises at least 2, 3, 4, 5, 6, 7, 8, 9, or 10 mismatches to the exclusion sequence.

In some embodiments, the target polynucleotide is DNA. In some embodiments, the target nucleic acid is RNA. In some embodiments, the RNA is mRNA.

In some embodiments, the melting temperature of a complementary region between one or more or each activating primer of the first extension reaction and a region of a template barcoded polynucleotide is lower than the annealing temperature used during the first extension reaction. For example, the melting temperature of a complementary region between one or more or each activating primer of the first extension reaction and a region of a template barcoded polynucleotide can be from 1-30, 2-30, 3-30, 4-30, 5-30, 6-30, 7-30, 8-30, 9-30, 10-30, 11-30, 12-30, 13-30, 14-30, 15-30, 16-30, 17-30, 18-30, 19-30, 20-30, 21-30, 22-30, 23-30, 24-30, 25-30, 26-30, 27-30, or 28-30 degrees lower than the annealing temperature used during the first extension reaction. For example, the melting temperature of a complementary region between one or more or each activating primer of the first extension reaction and a region of a template barcoded polynucleotide of from 1-3, 1-4, 1-5, 1-6, 1-7, 1-8, 1-9, 1-10, 1-11, 1-12, 1-13, 1-14, 1-15, 1-16, 1-17, 1-18, 1-19, 1-20, 1-21, 1-22, 1-23, 1-24, 1-25, 1-26, 1-27, 1-28, 1-29, or 1-30 degrees lower than the annealing temperature used during the first extension reaction. For example, the melting temperature of a complementary region between one or more or each activating primer of the first extension reaction and a region of a template barcoded polynucleotide can be from 1-20, 2-20, 3-20, 4-20, 5-20, 6-20, 7-20, 8-20, 9-20, 10-20, 11-20, 12-20, 13-20, 14-20, 15-20, 16-20, 17-20, or 18-20 degrees lower than the annealing temperature used during the first extension reaction. For example, the melting temperature of a complementary region between one or more or each activating primer of the first extension reaction and a region of a template barcoded polynucleotide can be from 10-12, 10-13, 10-14, 10-15, 10-16, 10-17, 10-18, 10-19, 10-20, 10-21, 10-22, 10-23, 10-24, 10-25, 10-26, 10-27, 10-28, 10-29, or 10-30 degrees lower than the annealing temperature used during the first extension reaction. For example, the melting temperature of a complementary region between one or more or each activating primer of the first extension reaction and a region of a template barcoded polynucleotide can be at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 degrees lower than the annealing temperature used during the first extension reaction.

In some aspects, one or more or each activating primer of the first extension reaction comprises a short sequence overlap between the activating primer and the template. For example, one or more or each activating primer of the first extension reaction comprises a 3-10 base overlap between the activating primer and the template. For example, one or more or each activating primer of the first extension reaction comprises a 3, 4, 5, 6, 7, 8, 9, or 10 base overlap between the activating primer and the template. In some embodiments, one or more or each activating primer of the first extension reaction comprises a 3, 4, 5, 6, 7, 8, 9, or 10 base overlap between the activating primer and the template and the melting temperature of a complementary region between one or more or each activating primer of the first extension reaction and a region of a template barcoded polynucleotide of from 1-30, 2-30, 3-30, 4-30, 5-30, 6-30, 7-30, 8-30, 9-30, 10-30, 11-30, 12-30, 13-30, 14-30, 15-30, 16-30, 17-30, 18-30, 19-30, 20-30, 21-30, 22-30, 23-30, 24-30, 25-30, 26-30, 27-30, or 28-30 degrees lower than the annealing temperature used during the first extension reaction.

In some embodiments, one or more or each activating primer of the first extension reaction comprises a 3, 4, 5, 6, 7, 8, 9, or 10 base overlap between the activating primer and the melting temperature of a complementary region between one or more or each activating primer of the first extension reaction and a region of a template barcoded polynucleotide of from 1-3, 1-4, 1-5, 1-6, 1-7, 1-8, 1-9, 1-10, 1-11, 1-12, 1-13, 1-14, 1-15, 1-16, 1-17, 1-18, 1-19, 1-20, 1-21, 1-22, 1-23, 1-24, 1-25, 1-26, 1-27, 1-28, 1-29, or 1-30 degrees lower than the annealing temperature used during the first extension reaction. In some embodiments, one or more or each activating primer of the first extension reaction comprises a 3, 4, 5, 6, 7, 8, 9, or 10 base overlap between the activating primer and the melting temperature of a complementary region between one or more or each activating primer of the first extension reaction and a region of a template barcoded polynucleotide of from 1-20, 2-20, 3-20, 4-20, 5-20, 6-20, 7-20, 8-20, 9-20, 10-20, 11-20, 12-20, 13-20, 14-20, 15-20, 16-20, 17-20, or 18-20 degrees lower than the annealing temperature used during the first extension reaction. In some embodiments, one or more or each activating primer of the first extension reaction comprises a 3, 4, 5, 6, 7, 8, 9, or 10 base overlap between the activating primer and the melting temperature of a complementary region between one or more or each activating primer of the first extension reaction and a region of a template barcoded polynucleotide at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 degrees lower than the annealing temperature used during the first extension reaction.

In some aspects, one or more or each activating primer of the first extension reaction comprises an activating region comprising a sequence that is from 5-30, 6-30, 7-30, 8-30, 9-30, 10-30, 11-30, 12-30, 13-30, 14-30, 15-30, 16-30, 17-30, 18-30, 19-30, 20-30, 21-30, 22-30, 23-30, 24-30, 25-30, 26-30, 27-30, or 28-30 bases in length and that has at least one mismatch between an exclusionary region of a template barcoded polynucleotide comprising a sequence that is the same number of bases as the activating region. In some embodiments, one or more or each activating primer of the first extension reaction comprises an activating region comprising a sequence that is from 5-30, 6-30, 7-30, 8-30, 9-30, 10-30, 11-30, 12-30, 13-30, 14-30, 15-30, 16-30, 17-30, 18-30, 19-30, 20-30, 21-30, 22-30, 23-30, 24-30, 25-30, 26-30, 27-30, or 28-30 bases in length and that has 1, 2, 3, 4, 5, 6, 7, 8, 9, 10, 11, 12, 13, 14, or 15 base mismatches to an exclusionary region of a template barcoded polynucleotide comprising a sequence that is the same number of bases as the activating region. In some embodiments, one or more or each activating primer of the first extension reaction comprises an activating region comprising a sequence that is from 5-30, 6-30, 7-30, 8-30, 9-30, 10-30, 11-30, 12-30, 13-30, 14-30, 15-30, 16-30, 17-30, 18-30, 19-30, 20-30, 21-30, 22-30, 23-30, 24-30, 25-30, 26-30, 27-30, or 28-30 bases in length and that has a melting temperature this is at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 degrees lower than the annealing temperature used during the first extension reaction. In some embodiments, one or more or each activating primer of the first extension reaction comprises an activating region comprising a sequence that is from 5-30, 6-30, 7-30, 8-30, 9-30, 10-30, 11-30, 12-30, 13-30, 14-30, 15-30, 16-30, 17-30, 18-30, 19-30, 20-30, 21-30, 22-30, 23-30, 24-30, 25-30, 26-30, 27-30, or 28-30 bases in length and that has a melting temperature to an exclusionary region of a template barcoded polynucleotide comprising a sequence that is the same number of bases as the activating region that is at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 degrees lower than the annealing temperature used during the first extension reaction. In some embodiments, one or more or each activating primer of the first extension reaction comprises an activating region comprising a sequence that is from 5-30, 6-30, 7-30, 8-30, 9-30, 10-30, 11-30, 12-30, 13-30, 14-30, 15-30, 16-30, 17-30, 18-30, 19-30, 20-30, 21-30, 22-30, 23-30, 24-30, 25-30, 26-30, 27-30, or 28-30 bases in length, a melting temperature to an exclusionary region of a template barcoded polynucleotide comprising a sequence that is the same number of bases as the activating region that is at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 degrees lower than the annealing temperature used during the first extension reaction, and 1, 2, 3, 4, 5, 6, 7, 8, 9, 10, 11, 12, 13, 14, or 15 base mismatches to the exclusionary region of the template barcoded polynucleotide.

In some aspects, a method comprises extending a second primer on the activated barcoded polynucleotide produced from the first extension reaction. In some aspects, a method comprises performing a second extension reaction comprising extending a second primer on the activated barcoded polynucleotide produced from the first extension reaction.

In some embodiments, the second primer has a difference from the melting temperature to an activated barcoded polynucleotide to the annealing temperature used during the second extension reaction that is less than a difference value from the melting temperature of the activating primer to the template barcoded polynucleotide of the first reaction. In some embodiments, the second primer has a difference from the melting temperature to an activated barcoded polynucleotide to the annealing temperature used during the second extension reaction that is less than a difference value from the melting temperature of the activating primer to an exclusionary region of a template barcoded polynucleotide.

In some embodiments, the second primer is an efficient extension primer. For example, the second primer can have a 80%-100% rate of extension initiation in the second extension reaction. In some embodiments, the second primer is an efficient amplification primer. For example, the second primer can have a 80%-100% rate of extension initiation in the first extension reaction. Where the second PCR primer is designed to work efficiently on the activated template, an activated template will be exponentially amplified in subsequent PCR cycles with efficiency close to 100%, i.e. doubling in number each cycle. In this situation, a 7-cycle difference between activation of the first and second barcoding templates within a droplet will mean that the products of the first activated barcode will outnumber products of the second barcode by -100 to 1, meaning that >~99% of products will contain the sequence of the first activated barcoding template. PCR amplification eventually plateaus and stops due to the phenomenon of product-reannealing, in which amplified product strands anneal to each other rather than to the primers. In embodiments where the template barcoded polynucleotides contain the same primer sequences, plateau-phase product reannealing of the first activated barcoded polynucleotide produced in a vessel can prevent or inhibit extension on or amplification of one or more or all other barcodes from all other template barcoded polynucleotides within the droplet. For example, were another primer to extend a second template barcoded polynucleotide after *(e.g.,* in a later amplification cycle) the formation of the first activated barcoded polynucleotide; the later activated barcodes will not be substantially copied or amplified (i.e. will never be able to "catch up" with copies of the first activated barcode). Thus, in some embodiments, the methods and compositions described herein can be used to exclude later activated barcodes from downstream sequence analysis and prevent their use when barcoding target polynucleotides (e.g., cell polynucleotides such as RNA or DNA in a vessel). In other words, through use of the methods and compositions described herein, the vast majority of DNA molecules capable of barcoding target polynucleotides from a cell within a droplet will be of a single sequence type.

In some aspects, a method comprises performing amplification after the first extension reaction. In some aspects, a method comprises performing amplification after the second extension reaction. In some aspects, a method comprises performing at least 2 PCR cycles. In some aspects, a method comprises performing at least 2 PCR cycles after a first extension reaction takes place. In some aspects, a method comprises performing at least 2 PCR cycles after a second extension reaction takes place. In some embodiments, a method comprises performing from 2-200 PCR cycles. In some embodiments, a method comprises performing from 2-100, 2-75, 2-60, 2-50, 2-40, 2-30, or 2-20 PCR cycles.

In some embodiments, a method comprises performing 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75, 80, 85, 90, 95, or 100 or more PCR cycles. In some embodiments, a first extension reaction has not occurred in one or more or each droplet of a plurality of droplets after one or more PCR cycles. In some embodiments, a first extension reaction has not occurred in one or more or each droplet of a plurality of droplets after 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75, 80, 85, 90, 95, or 100 or more PCR cycles.

### DROPLET GENERATION

Splitting a sample of a plurality of cells into small reaction volumes, coupled vessel barcoding of polynucleotides from, or derived from, an individual cell from the plurality of cells can enable high throughput sequencing of a repertoire of sequences, such as biomarker sequences.

Splitting a sample of a plurality of cells into small reaction volumes, coupled with vessel barcoding of polynucleotides from, or derived from, an individual cell from the plurality of cells can enable high throughput sequencing of a repertoire of sequences, such as sequences representing a percentage of the transcriptome of an organism. For example, a repertoire of sequences can comprise a plurality of sequences representing at least about 0.00001%, 0.00005%, 0.00010%, 0.00050%, 0.001%, 0.005%, 0.01%, 0.05%, 0.1%, 0.5%, 1%, 2%, 2.5%, 3%, 3.5%, 4%, 4.5%, 5%, 6%, 7%, 8%, 9%, 10%, 15%, 20%, 30%, 35%, 40%, 45, 50%, 55%, 60%, 65%, 70%, 75%, 80%, 85%, 90%, 95%, 98%, 99%, or 100% of the transcriptome of an organism.

Splitting a sample of immune cells into small reaction volumes, coupled with vessel barcoding of polynucleotides from, or derived from, an individual immune cell from the plurality of immune cells can enable high throughput sequencing of a repertoire of heavy and light chain sequences. These methods can also allow for pairing of the heavy and light chains after sequencing based on the barcoded sequences. Splitting a sample into small reaction volumes as described herein can also enable the use of reduced amounts of reagents, thereby lowering the material cost of the analysis.

In some cases, an extension or amplification reaction (e.g., PCR) are carried out in droplets, such as in droplet digital PCR. In certain aspects, the invention provides fluidic compartments to contain all or a portion of a target material. In some embodiments, a compartment is droplet. While reference is made to "droplets" throughout the specification, that term is used interchangeably with fluid compartment and fluid partition unless otherwise indicated. Except where indicated otherwise, "droplet" is used for convenience and any fluid partition or compartment may be used. The droplets used herein can include emulsion compositions (or mixtures of two or more immiscible fluids), such as described in US Patent No. 7,622,280. The droplets can be generated by devices described in WO/2010/036352. The term emulsion, as used herein, can refer to a mixture of immiscible liquids (such as oil and water). Oil-phase and/or water-in-oil emulsions allow for the compartmentalization of reaction mixtures within aqueous droplets. The emulsions can comprise aqueous droplets within a continuous oil phase. The emulsions provided herein can be oil-in-water emulsions, wherein the droplets are oil droplets within a continuous aqueous phase. The droplets provided herein are designed to prevent mixing between compartments, with each compartment protecting its contents from evaporation and coalescing with the contents of other compartments.

The mixtures or emulsions described herein can be stable or unstable. The emulsions can be relatively stable and have minimal coalescence. Coalescence occurs when small droplets combine to form progressively larger ones. In some cases, less than 0.00001%, 0.00005%, 0.00010%, 0.00050%, 0.001%, 0.005%, 0.01%, 0.05%, 0.1%, 0.5%, 1%, 2%, 2.5%, 3%, 3.5%, 4%, 4.5%, 5%, 6%, 7%, 8%, 9%, or 10% of droplets generated from a droplet generator coalesce with other droplets. The emulsions can also have limited flocculation, a process by which the dispersed phase comes out of suspension in flakes.

Droplets can be generated having an average diameter of about, less than about, or more than about, or at least about 0.001, 0.01, 0.05, 0.1, 1, 5, 10, 20, 30, 40, 50, 60, 70, 80, 100, 120, 130, 140, 150, 160, 180, 200, 300, 400, or 500 microns. Droplets can have an average diameter of about 0.001 to about 500, about 0.01 to about 500, about 0.1 to about 500, about 0.1 to about 100, about 0.01 to about 100, or about 1 to about 100 microns. Microfluidic methods of producing emulsion droplets using microchannel cross-flow focusing or physical agitation are known to produce either monodisperse or polydisperse emulsions. The droplets can be monodisperse droplets. The droplets can be generated such that the size of the droplets does not vary by more than plus or minus 5% of the average size of the droplets. In some cases, the droplets are generated such that the size of the droplets does not vary by more than plus or minus 2% of the average size of the droplets. A droplet generator can generate a population of droplets from a single sample, wherein none of the droplets vary in size by more than plus or minus about 0.1%, 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% of the average size of the total population of droplets.

Higher mechanical stability can be useful for microfluidic manipulations and higher-shear fluidic processing (*e.g*., in microfluidic capillaries or through 90 degree turns, such as valves, in fluidic path). Pre- and post-thermally treated droplets or capsules can be mechanically stable to standard pipet manipulations and centrifugation.

A droplet can be formed by flowing an oil phase through an aqueous sample. The aqueous phase can comprise a buffered solution and reagents for performing an amplification reaction, including cells, nucleotides, nucleotide analogues, molecular barcoded polynucleotides, vessel barcoded polynucleotides primers, template nucleic acids, and enzymes, such as a DNA polymerase, RNA polymerase, and/or reverse transcriptase.

The aqueous phase can comprise a buffered solution and reagents for performing an extension or amplification reaction with or without a solid surface, such as a bead. The buffered solution can comprise about, more than about, or less than about 1, 5, 10, 15, 20, 30, 50, 100, or 200 mM Tris. In some cases, the concentration of potassium chloride can be about, more than about, or less than about 10, 20, 30, 40, 50, 60, 80, 100, 200 mM. The buffered solution can comprise about 15 mM Tris and 50 mM KCl. The nucleotides can comprise deoxyribonucleotide triphosphate molecules, including dATP, dCTP, dGTP, and dTTP, in concentrations of about, more than about, or less than about 50, 100, 200, 300, 400, 500, 600, or 700 µm each. In some cases dUTP is added within the aqueous phase to a concentration of about, more than about, or less than about 50, 100, 200, 300, 400, 500, 600, or 700, 800, 900, or 1000 µm. In some cases, magnesium chloride or magnesium acetate (MgCl₂) is added to the aqueous phase at a concentration of about, more than about, or less than about 1.0, 2.0, 3.0, 4.0, or 5.0 mM. The concentration of MgCl₂ can be about 3.2 mM. In some cases, magnesium acetate or magnesium is used. In some cases, magnesium sulfate is used.

A non-specific blocking agent such as BSA or gelatin from bovine skin can be used, wherein the gelatin or BSA is present in a concentration range of approximately 0.1-0.9% w/v. Other possible blocking agents can include betalactoglobulin, casein, dry milk, or other common blocking agents. In some cases, preferred concentrations of BSA and gelatin are about 0.1% w/v.

Primers for extension or amplification within the aqueous phase can have a concentration of about, more than about, or less than about 0.05, 0.1, 0.2, 0.3, 0.4, 0.5, 0.6, 0.7, 0.8, 0.9, 1.0, 1.2, 1.5, 1.7, or 2.0 µm. Primer concentration within the aqueous phase can be about 0.05 to about 2, about 0.1 to about 1.0, about 0.2 to about 1.0, about 0.3 to about 1.0, about 0.4 to about 1.0, or about 0.5 to about 1.0µm. The concentration of primers can be about 0.5µm. Amenable ranges for target nucleic acid concentrations in PCR include, but are not limited to between about 1 pg and about 500 ng.

In some cases, the aqueous phase can also comprise additives including, but not limited to, nonspecific background/blocking nucleic acids (*e.g*., salmon sperm DNA), biopreservatives (*e.g.* sodium azide), PCR enhancers (*e.g.* Betaine, Trehalose, etc.), and inhibitors (*e.g.* RNAse inhibitors). Other additives can include, *e.g*., dimethyl sulfoxide (DMSO), glycerol, betaine (mono)hydrate (*N,N,N-*trimethylglycine = [caroxy-methyl] trimethylammonium), trehalose, 7-Deaza-2'-deoxyguanosine triphosphate (dC7GTP or 7-deaza-2'-dGTP), BSA (bovine serum albumin), formamide (methanamide), tetramethylammonium chloride (TMAC), other tetraalkylammonium derivatives (*e.g*., tetraethyammonium chloride (TEA-Cl) and tetrapropylammonium chloride (TPrA-Cl), non-ionic detergent (*e.g*., Triton X-100, Tween 20, Nonidet P-40 (NP-40)), or PREXCEL-Q. In some cases, the aqueous phase can comprise 0, 1, 2, 3, 4, 5, 6, 7, 8, 9, or 10 different additives. In other cases, the aqueous phase can comprise at least 0, 1, 2, 3, 4, 5, 6, 7, 8, 9, or 10 different additives.

In some cases, a non-ionic Ethylene Oxide/Propylene Oxide block copolymer can be added to the aqueous phase in a concentration of about 0.1%, 0.2%, 0.3%, 0.4%, 0.5%, 0.6%, 0.7%, 0.8%, 0.9%, or 1.0%. Common biosurfactants include non-ionic surfactants such as Pluronic F-68, Tetronics, and Zonyl FSN. Pluronic F-68 can be present at a concentration of about 0.5% w/v.

In some cases magnesium sulfate can be substituted for magnesium chloride, at similar concentrations. A wide range of common, commercial PCR buffers from varied vendors can be substituted for the buffered solution.

The emulsion can be formulated to produce highly monodisperse droplets having a liquid-like interfacial film that can be converted by heating into microcapsules having a solid-like interfacial film; such microcapsules can behave as bioreactors able to retain their contents through a reaction process such as extension or PCR amplification. The conversion to microcapsule form can occur upon heating. For example, such conversion can occur at a temperature of greater than about 50 °C, 60 °C, 70 °C, 80 °C, 90 °C, or 95 °C. In some cases this heating occurs using a thermocycler. During the heating process, a fluid or mineral oil overlay can be used to prevent evaporation. Excess continuous phase oil can or cannot be removed prior to heating. The biocompatible capsules can be resistant to coalescence and/or flocculation across a wide range of thermal and mechanical processing. Following conversion, the capsules can be stored at about, more than about, or less than about 3 °C, 4 °C, 5 °C, 6 °C, 7 °C, 8 °C, 9 °C 10 °C, 15 °C, 20 °C, 25 °C, 30 °C, 35 °C, or 40 °C. These capsules can be useful in biomedical applications, such as stable, digitized encapsulation of macromolecules, particularly aqueous biological fluids containing a mix of nucleic acids or protein, or both together; drug and vaccine delivery; biomolecular libraries; clinical imaging applications, and others.

The microcapsules can contain one or more polynucleotides and can resist coalescence, particularly at high temperatures. Accordingly, extension or PCR amplification reactions can occur at a very high density (e.g., number of reactions per unit volume). In some cases, greater than 100,000, 500,000, 1,000,000, 1,500,000, 2,000,000, 2,500,000, 5,000,000, or 10,000,000 separate reactions can occur per ml. In some cases, the reactions occur in a single well, *e.g*., a well of a microtiter plate, without inter-mixing between reaction volumes. The microcapsules can also contain other components necessary to enable a reverse transcription, primer extension, and/or PCR reaction to occur, *e.g*., primers, probes, dNTPs, DNA or RNA polymerases, etc. These capsules exhibit resistance to coalescence and flocculation across a wide range of thermal and mechanical processing.

In some cases, the extension or amplifying step is carried out by performing digital PCR, such as microfluidic-based digital PCR or droplet digital PCR.

Droplets can be generated using microfluidic systems or devices. As used herein, the "micro-" prefix (for example, as "microchannel" or "microfluidic"), generally refers to elements or articles having widths or diameters of less than about 1 mm, and less than about 100 microns (micrometers) in some cases. In some cases, the element or article includes a channel through which a fluid can flow. Additionally, "microfluidic", as used herein, refers to a device, apparatus or system that includes at least one microscale channel.

Microfluidic systems and devices have been described in a variety of contexts, typically in the context of miniaturized laboratory (*e.g*., clinical) analysis. Other uses have been described as well. For example, International Patent Application Publication Nos. WO 01/89788; WO 2006/040551; WO 2006/040554; WO 2004/002627; WO 2008/063227; WO 2004/091763; WO 2005/021151; WO 2006/096571; WO 2007/089541; WO 2007/081385 and WO 2008/063227.

A droplet generally includes an amount of a first sample fluid in a second carrier fluid. Any technique known in the art for forming droplets may be used with methods of the invention. An exemplary method involves flowing a stream of the sample fluid containing the target material (*e.g.*, immune cell) such that it intersects two opposing streams of flowing carrier fluid. The carrier fluid is immiscible with the sample fluid. Intersection of the sample fluid with the two opposing streams of flowing carrier fluid results in partitioning of the sample fluid into individual sample droplets containing the target material.

The carrier fluid may be any fluid that is immiscible with the sample fluid. An exemplary carrier fluid is oil. In certain embodiments, the carrier fluid includes a surfactant.

The same method may be applied to create individual droplets that contain other reagents such as reagents for an extension or amplification reaction such as a polymerase chain reaction (PCR), or a non-PCR based amplification reaction such as multi-strand displacement amplification, or other methods known to one of ordinary skill in the art. Suitable reagents for conducting PCR-based amplification reactions are known to those of ordinary skill in the art and include, but are not limited to, DNA polymerases, forward and reverse primers, deoxynucleotide triphosphates (dNTPs), and one or more buffers.

In certain embodiments, fluidic compartments are formed by providing a first fluid partition (*e.g*., a droplet) comprising a target material (*e.g*., an immune cell and/or a solid support such as a bead) and a second fluid (*e.g*., as a fluid stream or within droplets). The first and second fluids are merged to form a droplet. Merging can be accomplished by application of an electric field to the two fluids. In certain embodiments, the second fluid contains reagents for conducting an extension or amplification reaction, such as a polymerase chain reaction.

In certain aspects, the invention provides a method of making a library of uniquely barcoded heavy and light chain antibody sequences and/or alpha and beta chain TCR sequences and/or gamma and delta chain TCR sequences including obtaining a plurality of nucleic acid constructs in which each construct includes a unique N-mer and a functional N-mer. The functional N-mer can be a random N-mer, a PCR primer, a universal primer, an antibody, a sticky end, or any other sequence. The method can include making M sets of a number N of fluid compartments each containing one or more copies of a unique construct. The method can create barcode libraries of higher complexity by adding an additional construct to each compartment in a set, and repeating that for each set to produce N×M compartments each containing a unique pair of constructs. The pairs can be hybridized or ligated to produce new constructs. In each construct in a barcode library, each unique N-mer can be adapted for identification by sequencing, probe hybridization, other methods, or a combination of methods.

### DROPLET LIBRARIES

In general, a droplet library is made up of a number of library elements that are pooled together in a single collection. Libraries may vary in complexity from a single library element to 1×10¹⁵ library elements or more. Each library element is one or more given components at a fixed concentration. The element may be, but is not limited to, cells, beads, amino acids, proteins, polypeptides, nucleic acids, polynucleotides or small molecule chemical compounds. The element may contain an identifier such as a molecular barcode, a vessel barcode, or both.

A cell library element can include, but is not limited to, hybridomas, B-cells, T-cells, primary cells, cultured cell lines, cancer cells, stem cells, or any other cell type. Cellular library elements are prepared by encapsulating a number of cells from one to tens of thousands in individual droplets. The number of cells encapsulated is usually given by Poisson statistics from the number density of cells and volume of the droplet. However, in some cases the number deviates from Poisson statistics as described in Edd et al., "Controlled encapsulation of single-cells into monodisperse picolitre drops." Lab Chip, 8(8): 1262-1264, 2008. The discreet nature of cells allows for libraries to be prepared in mass with a plurality of cell variants, such as immune cells producing one antibody or TCR each, all present in a single starting media and then that media is broken up into individual droplet capsules that contain at most one cell. The cells within the individual droplets capsules are then lysed, heavy chain and light chain polynucleotides and/or alpha and beta chain polynucleotides and/or gamma and delta chain polynucleotides from the lysed cells are barcoded with molecular barcodes and vessel barcodes and amplified and then combined or pooled to form a library consisting of heavy and light chain and/or alpha and beta chain and/or gamma and delta chain library elements.

A bead based library element contains one or more beads, and may also contain other reagents, such as antibodies, enzymes or other proteins. In the case where all library elements contain different types of beads, but the same surrounding media, the library elements can all be prepared from a single starting fluid or have a variety of starting fluids. In the case of cellular libraries prepared in mass from a collection of variants, the library elements will be prepared from a variety of starting fluids. It is desirable to have exactly one cell per droplet with only a few droplets containing more than one cell when starting with a plurality of cells. In some cases, variations from Poisson statistics can be achieved to provide an enhanced loading of droplets such that there are more droplets with exactly one cell per droplet and few exceptions of empty droplets or droplets containing more than one cell.

In some embodiments, it is desirable to have exactly one vessel barcoded polynucleotide per droplet with only a few droplets containing more than one vessel barcoded polynucleotide when starting with a plurality of vessel barcoded polynucleotide. In some cases, variations from Poisson statistics can be achieved to provide an enhanced loading of droplets such that there are more droplets with exactly one vessel barcoded polynucleotide per droplet and few exceptions of empty droplets or droplets containing more than one vessel barcoded polynucleotide.

Examples of droplet libraries are collections of droplets that have different contents, ranging from beads, cells, small molecules, DNA, primers, antibodies, and barcoded polynucleotides. The droplets range in size from roughly 0.5 micron to 500 micron in diameter, which corresponds to about 1 picoliter to 1 nanoliter. However, droplets can be as small as 5 microns and as large as 500 microns. Preferably, the droplets are at less than 100 microns, about 1 micron to about 100 microns in diameter. The most preferred size is about 20 to 40 microns in diameter (10 to 100 picoliters). The preferred properties examined of droplet libraries include osmotic pressure balance, uniform size, and size ranges.

The droplets comprised within the droplet library provided by the instant invention are preferably uniform in size. That is, the diameter of any droplet within the library will vary less than 5%, 4%, 3%, 2%, 1% or 0.5% when compared to the diameter of other droplets within the same library. The uniform size of the droplets in the library may be critical to maintain the stability and integrity of the droplets and also may be essential for the subsequent use of the droplets within the library for the various biological and chemical assays described herein.

The invention provides a droplet library comprising a plurality of aqueous droplets within an immiscible fluid, wherein each droplet is preferably substantially uniform in size and comprises a different library element. The invention provides a method for forming the droplet library comprising providing a single aqueous fluid comprising different library elements, encapsulating each library element into an aqueous droplet within an immiscible fluid.

In certain embodiments, different types of elements (*e.g*., cells or beads), are pooled in a single source contained in the same medium. After the initial pooling, the elements are then encapsulated in droplets to generate a library of droplets wherein each droplet with a different type of bead or cell is a different library element. The dilution of the initial solution enables the encapsulation process. In some embodiments, the droplets formed will either contain a single element or will not contain anything, i.e., be empty. In other embodiments, the droplets formed will contain multiple copies of a library element. The elements being encapsulated are generally variants of a type. In one example, elements are immune cells of a blood sample, and each immune cell is encapsulated to amplify and barcode the antibody sequences of the nucleotides in the immune cells.

For example, in one type of emulsion library, there are library elements that have different particles, i.e., cells or barcoded polynucleotides in a different medium and are encapsulated prior to pooling. In one example, a specified number of library elements, i.e., n number of different cells or barcoded polynucleotides, is contained within different mediums. Each of the library elements are separately emulsified and pooled, at which point each of the n number of pooled different library elements are combined and pooled into a single pool. The resultant pool contains a plurality of water-in-oil emulsion droplets each containing a different type of particle.

In some embodiments, the droplets formed will either contain a single library element or will not contain anything, i.e., be empty. In other embodiments, the droplets formed will contain multiple copies of a library element. The contents of the beads follow a Poisson distribution, where there is a discrete probability distribution that expresses the probability of a number of events occurring in a fixed period of time if these events occur with a known average rate and independently of the time since the last event. The oils and surfactants used to create the libraries prevent the exchange of the contents of the library between droplets.

### AMPLIFICATION

The sample containing the target polynucleotide can comprise mRNA, or fragments thereof, which can be amplified. In some cases, the average length of the mRNA, or fragments thereof, can be less than about 100, 200, 300, 400, 500, or 800 base pairs, or less than about 5, 10, 20, 30, 40, 50, 60, 70, 80, 90, 100, 110, 120, 130, 140, 150, 160, 170, 180, 190, or 200 nucleotides, or less than about 1, 2, 5, 10, 20, 30, 40, 50, 60, 70, 80, 90, 100 kilobases. In some cases, a target sequence from a relative short template, such as a sample containing a template that is about 40, 45, 50, 55, 60, 65, 70, 75, 80, 85, 90, 95, or 100 bases, is amplified.

Thermocycling reactions can be performed on samples contained in reaction volumes (e.g., droplets). Droplets can be polydisperse or preferably monodisperse, generated through agitation, sonication or microfluidically through a T-channel junction or other means by those familiar with the art. Densities can exceed 20,000 droplets/40ul (1 nL droplets), 200,000 droplets/40ul (100 pL droplets). The droplets can remain intact during thermocycling. Droplets can remain intact during thermocycling at densities of greater than about 10,000 droplets/µL, 100,000 droplets/µL, 200,000 droplets/pL, 300,000 droplets/pL, 400,000 droplets/µL, 500,000 droplets/µL, 600,000 droplets/µL, 700,000 droplets/µL, 800,000 droplets/pL, 900,000 droplets/pL or 1,000,000 droplets/pL. In other cases, two or more droplets do not coalesce during thermocycling. In other cases, greater than 100 or greater than 1,000 droplets do not coalesce during thermocycling.

Any DNA polymerase that catalyzes primer extension can be used, including but not limited to E. coli DNA polymerase, Klenow fragment of E. coli DNA polymerase 1, T7 DNA polymerase, T4 DNA polymerase, Taq polymerase, Pfu DNA polymerase, Vent DNA polymerase, bacteriophage 29, REDTaq^{™}, Genomic DNA polymerase, or sequenase. In some cases, a thermostable DNA polymerase is used. A hot start PCR can also be performed wherein the reaction is heated to 95°C for two minutes prior to addition of the polymerase or the polymerase can be kept inactive until the first heating step in cycle 1. Hot start PCR can be used to minimize nonspecific extension or amplification. Any number of PCR cycles can be used to extend or amplify the target polynucleotide, e.g., about, more than about, or less than about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44 or 45 cycles. The number of amplification cycles can be about 1-45, 10-45, 20-45, 30-45, 35-45, 10-40, 10-30, 10-25, 10-20, 10-15, 20-35, 25-35, 30-35, or 35-40.

Amplification of target nucleic acids can be performed by any means known in the art. Target nucleic acids can be amplified by polymerase chain reaction (PCR) or isothermal DNA amplification. Examples of PCR techniques that can be used include, but are not limited to, quantitative PCR, quantitative fluorescent PCR (QF-PCR), multiplex fluorescent PCR (MF-PCR), real time PCR (reverse transcription-PCR), single cell PCR, restriction fragment length polymorphism PCR (PCR-RFLP), PCR-RFLP/reverse transcription-PCR-RFLP, hot start PCR, nested PCR, in situ polony PCR, in situ rolling circle amplification (RCA), digital PCR (dPCR), droplet digital PCR (ddPCR), bridge PCR, picoliter PCR and emulsion PCR. Other suitable amplification methods include the ligase chain reaction (LCR), transcription amplification, molecular inversion probe (MIP) PCR, self-sustained sequence replication, selective amplification of target polynucleotide sequences, consensus sequence primed polymerase chain reaction (CP-PCR), arbitrarily primed polymerase chain reaction (AP-PCR), degenerate polynucleotideprimed PCR (DOP-PCR) and nucleic acid based sequence amplification (NABSA). Other amplification methods that can be used herein include those described in U.S. Pat. Nos. 5,242,794; 5,494,810; 4,988,617; and 6,582,938, as well as include Q beta replicase mediated RNA amplification. Amplification can be isothermal amplification, e.g., isothermal linear amplification.

In some embodiments, extension or amplification does not occur on a solid support. In some embodiments, amplification does not occur on a solid support in a droplet. In some embodiments, amplification does occur on a solid support when the amplification is not in a droplet.

An extension or amplification reaction can comprise one or more additives. In some cases, the one or more additives are dimethyl sulfoxide (DMSO), glycerol, betaine (mono)hydrate (N,N,N-trimethylglycine = [caroxy-methyl] trimethylammonium), trehalose, 7-Deaza-2'-deoxyguanosine triphosphate (dC7GTP or 7-deaza-2'-dGTP), BSA (bovine serum albumin), formamide (methanamide), tetramethylammonium chloride (TMAC), other tetraalkylammonium derivatives (e.g., tetraethyammonium chloride (TEA-Cl) and tetrapropylammonium chloride (TPrA-Cl), non-ionic detergent (e.g., Triton X-100, Tween 20, Nonidet P-40 (NP-40)), or PREXCEL-Q. In some cases, an extension or amplification reaction comprises 0, 1, 2, 3, 4, 5, 6, 7, 8, 9, or 10 different additives. In other cases, an extension or amplification reaction comprises at least 0, 1, 2, 3, 4, 5, 6, 7, 8, 9, or 10 different additives.

### REVERSE TRANSCRIPTION

In some cases, target DNA polynucleotides are prepared from an RNA by reverse transcription. In some cases, the target polynucleotides are prepared from a DNA by primer extension, such as using a polymerase.

In some aspects, the methods described herein can be used in coupled reverse transcription-PCR (reverse transcription-PCR). For example, reverse transcription and PCR can be carried out in two distinct steps. First a cDNA copy of the sample mRNA can be synthesized using either a polynucleotide dT primer, a sequence specific primer, a universal primer, or any primer described herein.

Reverse transcription and PCR can be carried out in a single closed vessel reaction. For example, three primers can be employed, one for reverse transcription and two for PCR. The primer for reverse transcription can bind to the mRNA 3' to the position of the PCR amplicon. Although not essential, the reverse transcription primer can include RNA residues or modified analogs such as 2'-O-methyl RNA bases, which will not form a substrate for RNase H when hybridized to the mRNA.

The temperature to carry out the reverse transcription reaction depends on the reverse transcriptase being used. In some cases, a thermostable reverse transcriptase is used and the reverse transcription reaction is carried out at about 37 °C to about 75 °C, at about 37 °C to about 50 °C, at about 37 °C to about 55 °C, at about 37 °C to about 60 °C, at about 55 °C to about 75 °C, at about 55 °C to about 60 °C, at about 37 °C, or at about 60 °C. In some cases, a reverse transcriptase that transfers 3 or more non-template terminal nucleotides to an end of the transcribed product is used.

A reverse transcription reaction and the PCR reaction described herein can be carried out in various formats known in the art, such as in tubes, microtiter plates, microfluidic devices, or, preferably, droplets.

A reverse transcription reaction can be carried out in volumes ranging from 5 µL to 100 µL, or in 10 µL to 20 µL reaction volumes. In droplets, reaction volumes can range from 1 pL to 100 nL, or 10 pL to 1 nL. In some cases, the reverse transcription reaction is carried out in a droplet having a volume that is about or less than 1 nL. In some cases, a PCR reaction is in a droplet having a reaction volume ranges from 1 pL to 100 nL preferably 10 pL to 1 nL. In some cases, the PCR reaction is carried out in a droplet having a volume that is about or less than 1 nL. In some cases, a reverse transcription reaction and a PCR reaction are carried out in the same droplet having a reaction volume ranges from 1 pL to 100 nL or 10 pL to 1 nL. In some cases, the reverse transcription reaction and the PCR reaction are carried out in a droplet having a volume that is about or less than 1 nL or a volume that is about or less than 1 pL. In some cases, a reverse transcription reaction and a PCR reaction are carried out in a different droplet. In some cases, a reverse transcription reaction and a PCR reaction are carried out in a plurality of droplets each having a reaction volume ranges from 1 pL to 100 nL or 10 pL to 1 nL. In some cases, the reverse transcription reaction and the PCR reaction are carried out in a plurality of droplets each having a volume that is about or less than 1 nL.

Target polynucleotides, such as RNA, can be reverse transcribed into cDNA using one or more reverse transcription primers. The one or more reverse transcription primers can comprise a region complementary to a region of the RNA, such as a constant region *(e.g.,* a heavy or light chain constant region or a poly-A tail of mRNA). In some embodiments, the reverse transcription primers can comprise a first reverse transcription primer with a region complementary to a constant region of a first RNA, and a second reverse transcription primer with a region complementary to a constant region of a second RNA. In some embodiments, the reverse transcription primers can comprise a first reverse transcription primer with a region complementary to a constant region of a first RNA, and one or more reverse transcription primers with a region complementary to a constant region of one or more RNAs, respectively.

In some embodiments, reverse transcription primers do not comprise a barcode.

Reverse transcription primers can further comprise a region that is not complementary to a region of the RNA. In some embodiments, the region that is not complementary to a region of the RNA is 5' to a region of the primers that is complementary to the RNA. In some embodiments, the region that is not complementary to a region of the RNA is 3' to a region of the primers that is complementary to the RNA. In some embodiments, the region that is not complementary to a region of the RNA is a 5' overhang region. In some embodiments, the region that is not complementary to a region of the RNA comprises a priming site for amplification and/or a sequencing reaction. Using the one or more primers described herein, the RNA molecules are reverse transcribed using suitable reagents known in the art.

After performing the reverse transcription reactions of the RNA molecules, the resulting cDNA molecules can be barcoded with a molecular barcode and a vessel barcode and amplified by one or more PCR reactions, such as a first and/or a second PCR reaction. The first and/or second PCR reaction can utilize a pair of primers or a plurality of primer pairs. The first and/or second PCR reaction can utilize a plurality of forward/reverse primers and a reverse primer. The first and/or second PCR reaction can utilize a plurality of forward/reverse primers and a forward primer. A first and/or second primer of a plurality of forward/reverse primers can be a forward/reverse primer containing a region complementary to the cDNA molecules or barcoded cDNA molecules. A first and/or second primer of a plurality of forward/reverse primers can be a forward/reverse primer containing a region complementary to the barcoded cDNA molecules.

In some embodiments, a plurality of forward/reverse primers comprises one or more forward/reverse primers wherein each of the forward/reverse primers in the plurality of forward/reverse primers comprises a region complementary to one or more upstream or downstream regions to a V segment of the cDNAs or barcoded cDNAs. For example, a plurality of forward/reverse primers comprises a forward/reverse primer comprising a region complementary to a upstream or downstream region to a V segment of the cDNAs or barcoded cDNAs and one or more other forward/reverse primers comprising a region complementary to one or more other upstream or downstream regions to a V segment of the cDNAs or barcoded cDNAs. For example, a plurality of forward/reverse primers comprises a first and/or second forward/reverse primer comprising a region complementary to a first and/or second upstream or downstream region to a V segment of the cDNAs or barcoded cDNAs and a second forward/reverse primer comprising a region complementary to a second upstream or downstream region to a V segment of the cDNAs or barcoded cDNAs. For example, a plurality of forward/reverse primers comprises a first and/or second forward/reverse primer comprising a region complementary to a first and/or second upstream or downstream region to a V segment of the cDNAs or barcoded cDNAs, a second forward/reverse primer comprising a region complementary to a second upstream or downstream region to a V segment of the cDNAs or barcoded cDNAs, and a third forward/reverse primer comprising a region complementary to a third upstream or downstream region to a V segment of the cDNAs or barcoded cDNAs, etc. The primers in the plurality of forward/reverse primers can be used to anneal to all possible upstream or downstream regions of all V segments expressed by the cells, such as immune B-cells or T-cells, in the sample.

In some embodiments, a plurality of forward/reverse primers comprises one or more forward/reverse primers wherein each of the forward/reverse primers in the plurality of forward/reverse primers comprises a region complementary to one or more upstream or downstream regions to a C segment of the cDNAs or barcoded cDNAs. For example, a plurality of forward/reverse primers comprises a forward/reverse primer comprising a region complementary to a upstream or downstream region to a C segment of the cDNAs or barcoded cDNAs and one or more other forward/reverse primers comprising a region complementary to one or more other upstream or downstream regions to a C segment of the cDNAs or barcoded cDNAs. For example, a plurality of forward/reverse primers comprises a first and/or second forward/reverse primer comprising a region complementary to a first and/or second upstream or downstream region to a C segment of the cDNAs or barcoded cDNAs and a second forward/reverse primer comprising a region complementary to a second upstream or downstream region to a C segment of the cDNAs or barcoded cDNAs. For example, a plurality of forward/reverse primers comprises a first and/or second forward/reverse primer comprising a region complementary to a first and/or second upstream or downstream region to a C segment of the cDNAs or barcoded cDNAs, a second forward/reverse primer comprising a region complementary to a second upstream or downstream region to a C segment of the cDNAs or barcoded cDNAs, and a third forward/reverse primer comprising a region complementary to a third upstream or downstream region to a C segment of the cDNAs or barcoded cDNAs, etc. The primers in the plurality of forward/reverse primers can be used to anneal to all possible upstream or downstream regions of all C segments expressed by the cells, such as immune B-cells or T-cells, in the sample.

In some embodiments, a plurality of forward/reverse primers comprises one or more forward/reverse primers wherein each of the forward/reverse primers in the plurality of forward/reverse primers comprises a region complementary to one or more upstream or downstream regions to a molecular barcode of the barcoded cDNAs. For example, a plurality of forward/reverse primers comprises a forward/reverse primer comprising a region complementary to a upstream or downstream region to a molecular barcode of the barcoded cDNAs and one or more other forward/reverse primers comprising a region complementary to one or more other upstream or downstream regions to a molecular barcode of the barcoded cDNAs. For example, a plurality of forward/reverse primers comprises a first and/or second forward/reverse primer comprising a region complementary to a first and/or second upstream or downstream region to a molecular barcode of the barcoded cDNAs and a second forward/reverse primer comprising a region complementary to a second upstream or downstream region to a molecular barcode of the barcoded cDNAs. For example, a plurality of forward/reverse primers comprises a first and/or second forward/reverse primer comprising a region complementary to a first and/or second upstream or downstream region to a molecular barcode of the barcoded cDNAs, a second forward/reverse primer comprising a region complementary to a second upstream or downstream region to a molecular barcode of the barcoded cDNAs, and a third forward/reverse primer comprising a region complementary to a third upstream or downstream region to a molecular barcode of the barcoded cDNAs, etc. The plurality of forward/reverse primers can be used to anneal to all possible upstream or downstream regions of all molecular barcodes expressed by the cells, such as immune B-cells or T-cells, in the sample.

In some embodiments, a plurality of forward/reverse primers comprises one or more forward/reverse primers wherein each of the forward/reverse primers in the plurality of forward/reverse primers comprises a region complementary to one or more upstream or downstream regions to a vessel barcode of the barcoded cDNAs. For example, a plurality of forward/reverse primers comprises a forward/reverse primer comprising a region complementary to a upstream or downstream region to a vessel barcode of the barcoded cDNAs and one or more other forward/reverse primers comprising a region complementary to one or more other upstream or downstream regions to a vessel barcode of the barcoded cDNAs. For example, a plurality of forward/reverse primers comprises a first and/or second forward/reverse primer comprising a region complementary to a first and/or second upstream or downstream region to a vessel barcode of the barcoded cDNAs and a second forward/reverse primer comprising a region complementary to a second upstream or downstream region to a vessel barcode of the barcoded cDNAs. For example, a plurality of forward/reverse primers comprises a first and/or second forward/reverse primer comprising a region complementary to a first and/or second upstream or downstream region to a vessel barcode of the barcoded cDNAs, a second forward/reverse primer comprising a region complementary to a second upstream or downstream region to a vessel barcode of the barcoded cDNAs, and a third forward/reverse primer comprising a region complementary to a third upstream or downstream region to a vessel barcode of the barcoded cDNAs, etc. The primers in the plurality of forward/reverse primers can be used to anneal to all possible upstream or downstream regions of all vessel barcodes expressed by the cells, such as immune B-cells or T-cells, in the sample.

The forward/reverse primers in the plurality of forward/reverse primers further comprise a region that is not complementary to a region of the RNA. In some embodiments, the region that is not complementary to a region of the RNA is 5' to a region of the forward/reverse primers that is complementary to the RNA (i.e. a upstream or downstream regions of a V segment). In some embodiments, the region that is not complementary to a region of the RNA is 3' to a region of the forward/reverse primers that is complementary to the RNA. In some embodiments, the region that is not complementary to a region of the RNA is a 5' overhang region. In some embodiments, the region that is not complementary to a region of the RNA comprises a priming site for amplification and/or a second sequencing reaction. In some embodiments, the region that is not complementary to a region of the RNA comprises a priming site for amplification and/or a third sequencing reaction. In some embodiments, the region that is not complementary to a region of the RNA comprises a priming site for a second and a third sequencing reaction. In some embodiments, the sequence of the priming site for the second and the third sequencing reaction are the same. Using the one or more forward/reverse primers and a reverse primer as described herein, the cDNA molecules are amplified using suitable reagents known in the art. In some embodiments, a region is complementary to a region of the RNA, such as the constant region or a poly-A tail of mRNA.

In some embodiments, the method further comprises an extension primer blocking oligonucleotide. For example, the method can comprise performing a reverse transcription reaction in the presence of an extension primer blocking oligonucleotide. In some embodiments, the reverse transcription reaction comprises reverse transcribing the target polynucleotide. In some embodiments, the plurality of vessels comprises an extension primer blocking oligonucleotide. In some embodiments, the extension primer blocking oligonucleotide prevents extension of the extension primer or amplification primer before PCR. In some embodiments, the extension primer blocking oligonucleotide prevents extension of the extension primer or amplification primer during reverse transcription.

In some embodiments, the reverse transcription reaction is before the amplifying or the amplification reaction. In some embodiments, the reverse transcription reaction is before the barcoding. In some embodiments, the reverse transcription reaction is before the first extension reaction. In some embodiments, the reverse transcription reaction is before the second extension reaction. In some embodiments, the reverse transcription reaction is after the forming a plurality of vessels.

In some embodiments, the extension primer blocking oligonucleotide has a melting temperature to a template barcoded polynucleotide of the plurality of template barcoded polynucleotides that is higher than the melting temperature of an extension primer or amplification primer to the template barcoded polynucleotide of the plurality of template barcoded polynucleotides. In some embodiments, the extension primer blocking oligonucleotide has a melting temperature to a template barcoded polynucleotide of the plurality of template barcoded polynucleotides that is at least 1 °C, 2 °C, 3 °C, 4 °C, 5 °C, 6 °C, 7 °C, 8 °C, 9 °C, 10 °C, 11 °C, 12 °C, 13 °C, 14 °C, 15 °C, 16 °C, 17 °C, 18 °C, 19 °C, 20 °C, 21 °C, 22 °C, 23 °C, 24 °C, 25 °C, 26 °C, 27 °C, 28 °C, 29 °C, or 30 °C higher than the melting temperature of an extension primer or amplification primer to the template barcoded polynucleotide of the plurality of template barcoded polynucleotides. In some embodiments, the extension primer blocking oligonucleotide has a melting temperature to the plurality of template barcoded polynucleotides that is higher than the melting temperature of an extension primer or amplification primer to the plurality of template barcoded polynucleotides.

In some embodiments, the extension primer blocking oligonucleotide is annealed to the template barcoded polynucleotides during the reverse transcription reaction. In some embodiments, the extension primer blocking oligonucleotide is not annealed to the template barcoded polynucleotides during the amplifying or the amplification reaction, such as PCR.

In some embodiments, the extension primer or amplification primer hybridizes to a region of the template barcoded polynucleotides that hybridizes to the extension primer blocking oligonucleotide. In some embodiments, the region of the template barcoded polynucleotides that hybridizes to the extension primer or amplification primer is shorter than a region of the template barcoded polynucleotide to which the extension primer blocking oligonucleotide hybridizes. In some embodiments, the extension primer or amplification primer hybridizes to a first region of the template barcoded polynucleotides and the extension primer blocking oligonucleotide hybridizes to a second region of the template barcoded polynucleotides, wherein the first region and the second region overlap.

In some embodiments, the extension primer blocking oligonucleotide is longer than the first extension primer. In some embodiments, the extension primer blocking oligonucleotide hybridizes to a region of the plurality of template barcoded polynucleotides comprising the exclusionary sequence. In some embodiments, the region of the plurality of template barcoded polynucleotides comprising the exclusionary sequence to which the extension primer blocking oligonucleotide hybridizes is longer than the exclusionary sequence.

### PRIMERS

Generally, one or more pairs of primers can be used in an amplification reaction; one primer of a primer pair can be a forward primer and one primer of a primer pair can be a reverse primer.

In some cases, a first pair of primers can be used in the amplification reaction; one primer of the first pair can be a forward primer complementary to a sequence of a first target polynucleotide molecule and one primer of the first pair can be reverse primer can be complementary to a second sequence of the first target polynucleotide molecule, and a first target locus can reside between the first sequence and the second sequence. In some embodiments, the first target locus comprises a VH or Vα or Vγ sequence.

In some cases, a second pair of primers can be used in the amplification reaction; one primer of the second pair can be a forward primer complementary to a first sequence of a second target polynucleotide molecule and one primer of the second pair can be a reverse primer complementary to a second sequence of the second target polynucleotide molecule, and a second target locus can reside between the first sequence and the second sequence. In some embodiments, the second target locus comprises a VL or Vβ or Vδ sequence.

In some cases, a third pair of primers can be used in the amplification reaction; one primer of the third pair can be a forward primer complementary to a first sequence of a third target polynucleotide molecule and one primer of the third pair can be a reverse primer complementary to a second sequence of the third target polynucleotide molecule, and a third target locus can reside between the first sequence and the second sequence. In some embodiments, the third target locus comprises a barcode, such as a molecular barcode or vessel barcode.

The length of the forward primer and the reverse primer can depend on the sequence of the target polynucleotide and the target locus. For example, the length and/or TM of the forward primer and reverse primer can be optimized. In some case, a primer can be about, more than about, or less than about 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 nucleotides in length. In some cases, a primer is about 15 to about 20, about 15 to about 25, about 15 to about 30, about 15 to about 40, about 15 to about 45, about 15 to about 50, about 15 to about 55, about 15 to about 60, about 20 to about 25, about 20 to about 30, about 20 to about 35, about 20 to about 40, about 20 to about 45, about 20 to about 50, about 20 to about 55, or about 20 to about 60 nucleotides in length.

A primer can be a single-stranded DNA prior to binding a template polynucleotide. In some cases, the primer initially comprises double-stranded sequence. The appropriate length of a primer can depend on the intended use of the primer but can range from about 6 to about 50 nucleotides, or from about 15 to about 35 nucleotides. Short primer molecules can generally require cooler temperatures to form sufficiently stable hybrid complexes with a template. In some embodiments, a primer need not reflect the exact sequence of the template nucleic acid, but can be sufficiently complementary to hybridize with a template. In some cases, a primer can be partially double-stranded before binding to a template polynucleotide. A primer with double-stranded sequence can have a hairpin loop of about, more than about, or less than about 3, 4, 5, 6, 7, 8, 9, 10, 11, 12, 13, 14, 15, 16, 17, 18, 19, or 20 bases. A double stranded portion of a primer can be about, more than about, less than about, or at least about 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 base-pairs.

Primers can incorporate additional features that allow for the detection or immobilization of the primer but do not alter a basic property of the primer (e.g., acting as a point of initiation of DNA synthesis). For example, primers can contain an additional nucleic acid sequence at the 5' end which does not hybridize to a target nucleic acid, but which facilitates cloning or further amplification, or sequencing of an amplified product. For example, the additional sequence can comprise a primer binding site, such as a universal primer binding site. A region of the primer which is sufficiently complementary to a template to hybridize can be referred to herein as a hybridizing region.

In another case, a primer utilized in methods and compositions described herein can comprise one or more universal nucleosides. Non-limiting examples of universal nucleosides are 5-nitroindole and inosine, as described in U.S. Appl. Pub. Nos. 2009/0325169 and 2010/0167353.

Primers can be designed according to known parameters for avoiding secondary structures and self-hybridization. Different primer pairs can anneal and melt at about the same temperatures, for example, within 1 °C, 2 °C, 3 °C, 4 °C, 5 °C, 6 °C, 7 °C, 8 °C, 9 °C or 10 °C of another primer pair. In some cases, greater than 1, 2, 3, 4, 5, 6, 7, 8, 9, 10, 15, 20, 25, 30, 35, 40, 45, 50, 100, 200, 500, 1000, 5000, 10,000 or more primers are initially used. Such primers can hybridize to target polynucleotides described herein.

Primers can be prepared by a variety of methods including but not limited to cloning of appropriate sequences and direct chemical synthesis using methods well known in the art (Narang et al., Methods Enzymol. 68:90 (1979); Brown et al., Methods Enzymol. 68:109 (1979)). Primers can also be obtained from commercial sources. The primers can have an identical melting temperature. The primers can have non-identical melting temperatures. The lengths of the primers can be extended or shortened at the 5' end or the 3' end to produce primers with desired melting temperatures. One of the primers of a primer pair can be longer than the other primer. The 3' annealing lengths of the primers, within a primer pair, can differ. Also, the annealing position of each primer pair can be designed such that the sequence and length of the primer pairs yield the desired melting temperature. An equation for determining the melting temperature of primers smaller than 25 base pairs is the Wallace Rule (TM=2(A+T)+4(G+C)). Computer programs can also be used to design primers. The TM (melting or annealing temperature) of each primer can be calculated using software programs. The annealing temperature of the primers can be recalculated and increased after any cycle of amplification, including but not limited to cycle 1, 2, 3, 4, 5, cycles 6-10, cycles 10-15, cycles 15-20, cycles 20-25, cycles 25-30, cycles 30-35, or cycles 35-40. After the initial cycles of amplification, the 5' half of the primers can be incorporated into the products from each loci of interest; thus the TM can be recalculated based on both the sequences of the 5' half and the 3' half of each primer.

Conducting the one or more reactions of the methods disclosed herein can comprise the use of one or more primers. As used herein, a primer comprises a double-stranded, single-stranded, or partially single-stranded polynucleotide that is sufficiently complementary to hybridize to a template polynucleotide. A primer can be a single-stranded DNA prior to binding a template polynucleotide. In some embodiments, the primer initially comprises double-stranded sequence. A primer site includes the area of the template to which a primer hybridizes. In some embodiments, primers are capable of acting as a point of initiation for template-directed nucleic acid synthesis. For example, primers can initiate template-directed nucleic acid synthesis when four different nucleotides and a polymerization agent or enzyme, such as DNA or RNA polymerase or reverse transcriptase. A primer pair includes 2 primers: a first primer with a 5' upstream region that hybridizes with a 5' end of a template sequence, and a second primer with a 3' downstream region that hybridizes with the complement of the 3' end of the template sequence. A primer set includes two or more primers: a first primer or first plurality of primers with a 5' upstream region that hybridizes with a 5' end of a template sequence or plurality of template sequences, and a second primer or second plurality of primers with a 3' downstream region that hybridizes with the complement of the 3' end of the template sequence or plurality of template sequences. In some embodiments, a primer comprises a target specific sequence. In some embodiments, a primer comprises a sample barcode sequence. In some embodiments, a primer comprises a universal priming sequence. In some embodiments, a primer comprises a PCR priming sequence. In some embodiments, a primer comprises a PCR priming sequence used to initiate amplification of a polynucleotide. (Dieffenbach, PCR Primer: A Laboratory Manual, 2nd Edition (Cold Spring Harbor Press, New York (2003)). The universal primer binding site or sequence allows the attachment of a universal primer to a polynucleotide and/or amplicon. Universal primers are well known in the art and include, but are not limited to, -47F (M13F), alfaMF, AOX3', AOX5', BGHr, CMV-30, CMV-50, CVMf, LACrmt, lamgda gt10F, lambda gt 10R, lambda gt11F, lambda gt11R, M13 rev, M13Forward(-20), M13Reverse, male, p10SEQPpQE, pA-120, pet4, pGAP Forward, pGLRVpr3, pGLpr2R, pKLAC14, pQEFS, pQERS, pucU1, pucU2, reversA, seqIREStam, seqIRESzpet, seqori, seqPCR, seqpIRES-, seqpIRES+, seqpSecTag, seqpSecTag+, seqretro+PSI, SP6, T3-prom, T7-prom, and T7-termInv. As used herein, attach can refer to both or either covalent interactions and noncovalent interactions. Attachment of the universal primer to the universal primer binding site may be used for amplification, detection, and/or sequencing of the polynucleotide and/or amplicon. The universal primer binding site may comprise at least about 1, 2, 3, 4, 5, 6, 7, 8, 9, 10, 11, 12, 13, 14, 15, 16, 17, 18, 19, 20, 30, 40, 50, 60, 70, 80, 90, 100, 200, 300, 400, 500, 600, 700, 800, 900, or 1000 nucleotides or base pairs. In another example, the universal primer binding site comprises at least about 1500, 2000, 2500, 3000, 3500, 4000, 4500, 5000, 5500, 6000, 6500, 7000, 7500, 8000, 8500, 9000, 9500, or 10000 nucleotides or base pairs. In some embodiments, the universal primer binding site comprises 1-10, 10-20, 10-30 or 10-100 nucleotides or base pairs. In some embodiments, the universal primer binding site comprises from about 1-90, 1-80, 1-70, 1-60, 1-50, 1-40, 1-30, 1-20, 1-10, 2-90, 2-80, 2-70, 2-60, 2-50, 2-40, 2-30, 2-20, 2-10, 1-900, 1-800, 1-700, 1-600, 1-500, 1-400, 1-300, 1-200, 1-100, 2-900, 2-800, 2-700, 2-600, 2-500, 2-400, 2-300, 2-200, 2-100, 5-90, 5-80, 5-70, 5-60, 5-50, 5-40, 5-30, 5-20, 5-10, 10-90, 10-80, 10-70, 10-60, 10-50, 10-40, 10-30, 10-20, 10-10, 5-900, 5-800, 5-700, 5-600, 5-500, 5-400, 5-300, 5-200, 5-100, 10-900, 10-800, 10-700, 10-600, 10-500, 10-400, 10-300, 10-200, 10-100, 25-900, 25-800, 25-700, 25-600, 25-500, 25-400, 25-300, 25-200, 25-100, 100-1000, 100-900, 100-800, 100-700, 100-600, 100-500, 100-400, 100-300, 100-200, 200-1000, 200-900, 200-800, 200-700, 200-600, 200-500, 200-400, 200-300, 300-1000, 300-900, 300-800, 300-700, 300-600, 300-500, 300-400, 400-1000, 400-900, 400-800, 400-700, 400-600, 400-500, 500-1000, 500-900, 500-800, 500-700, 500-600, 600-1000, 600-900, 600-800, 600-700, 700-1000, 700-900, 700-800, 800-1000, 800-900, or 900-1000 nucleotides or base pairs.

Primers can have a length compatible with its use in synthesis of primer extension products. A primer can be a polynucleotide that is 8 to 200 nucleotides in length. The length of a primer can depend on the sequence of the template polynucleotide and the template locus. For example, the length and/or melting temperature (TM) of a primer or primer set can be optimized. In some case, a primer can be about, more than about, or less than about 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 nucleotides in length. In some embodiments, primers are about 8-100 nucleotides in length, for example, 10-75, 15-60, 15-40, 18-30, 20-40, 21-50, 22-45, 25-40, 7-9, 12-15, 15-20, 15-25, 15-30, 15-45, 15-50, 15-55, 15-60, 20-25, 20-30, 20-35, 20-45, 20-50, 20-55, or 20-60 nucleotides in length and any length there between. In some embodiments, primers are at most about 10, 12, 15, 20, 21, 22, 23, 24, 25, 26, 27, 28, 29, 30, 35, 40, 45, 50, 55, 60, 65, 70, 75, 80, 85, 90, 95 or 100 nucleotides in length.

Generally, one or more pairs of primers can be used in an exponential amplification reaction; one primer of a primer pair can be a forward primer and one primer of a primer pair can be a reverse primer. In some embodiments, a first pair of primers can be used in the exponential amplification reaction; one primer of the first pair can be a forward primer complementary to a sequence of a first template polynucleotide molecule and one primer of the first pair can be a reverse primer complementary to a second sequence of the first template polynucleotide molecule, and a first template locus can reside between the first sequence and the second sequence. In some embodiments, a second pair of primers can be used in the amplification reaction; one primer of the second pair can be a forward primer complementary to a first sequence of a second target polynucleotide molecule and one primer of the second pair can be a reverse primer complementary to a second sequence of the second target polynucleotide molecule, and a second target locus can reside between the first sequence and the second sequence. In some embodiments, the second target locus comprises a variable light chain antibody sequence. In some embodiments, a third pair of primers can be used in the amplification reaction; one primer of the third pair can be a forward primer complementary to a first sequence of a third template polynucleotide molecule and one primer of the third pair can be a reverse primer complementary to a second sequence of the third template polynucleotide molecule, and a third template locus can reside between the first sequence and the second sequence.

The one or more primers can anneal to at least a portion of a plurality of template polynucleotides. The one or more primers can anneal to the 3' end and/or 5' end of the plurality of template polynucleotides. The one or more primers can anneal to an internal region of the plurality of template polynucleotides. The internal region can be at least about 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 650, 700, 750, 800, 850, 900 or 1000 nucleotides from the 3' ends or 5' ends the plurality of template polynucleotides. The one or more primers can comprise a fixed panel of primers. The one or more primers can comprise at least one or more custom primers. The one or more primers can comprise at least one or more control primers. The one or more primers can comprise at least one or more housekeeping gene primers. The one or more primers can comprise a universal primer. The universal primer can anneal to a universal primer binding site. In some embodiments, the one or more custom primers anneal to an SBC, a target specific region, complements thereof, or any combination thereof. The one or more primers can comprise a universal primer. The one or more primers primer can be designed to amplify or perform primer extension, reverse transcription, linear extension, non-exponential amplification, exponential amplification, PCR, or any other amplification method of one or more target or template polynucleotides

The target specific region can comprise at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 650, 700, 750, 800, 850, 900 or 1000 nucleotides or base pairs. In another example, the target specific region comprises at least about 1500, 2000, 2500, 3000, 3500, 4000, 4500, 5000, 5500, 6000, 6500, 7000, 7500, 8000, 8500, 9000, 9500, or 10000 nucleotides or base pairs. in some embodiments, the target specific region comprises from about 5-10, 10-15, 10-20, 10-30, 15-30, 10-75, 15-60, 15-40, 18-30, 20-40, 21-50, 22-45, 25-40, 7-9, 12-15, 15-20, 15-25, 15-30, 15-45, 15-50, 15-55, 15-60, 20-25, 20-30, 20-35, 20-45, 20-50, 20-55, 20-60, 2-900, 2-800, 2-700, 2-600, 2-500, 2-400, 2-300, 2-200, 2-100, 25-900, 25-800, 25-700, 25-600, 25-500, 25-400, 25-300, 25-200, 25-100, 100-1000, 100-900, 100-800, 100-700, 100-600, 100-500, 100-400, 100-300, 100-200, 200-1000, 200-900, 200-800, 200-700, 200-600, 200-500, 200-400, 200-300, 300-1000, 300-900, 300-800, 300-700, 300-600, 300-500, 300-400, 400-1000, 400-900, 400-800, 400-700, 400-600, 400-500, 500-1000, 500-900, 500-800, 500-700, 500-600, 600-1000, 600-900, 600-800, 600-700, 700-1000, 700-900, 700-800, 800-1000, 800-900, or 900-1000 nucleotides or base pairs.

Primers can be designed according to known parameters for avoiding secondary structures and self-hybridization. In some embodiments, different primer pairs can anneal and melt at about the same temperatures, for example, within 1 °C, 2 °C, 3 °C, 4 °C, 5 °C, 6 °C, 7 °C, 8 °C, 9 °C or 10 °C of another primer pair. In some embodiments, one or more primers in a plurality of primers can anneal and melt at about the same temperatures, for example, within 1, 2, 3, 4, 5, 6, 7, 8, 9 or 10°C of another primer in the plurality of primers. In some embodiments, one or more primers in a plurality can anneal and melt at different temperatures than another primer in the plurality of primers.

A plurality of primers for one or more steps of the methods described herein can comprise a plurality of primers comprising about, at most about, or at least about 1, 2, 3, 4, 5, 6, 7, 8, 9, 10, 11, 12, 13, 14, 15, 16, 17, 18, 19, 20, 30, 40, 50, 60, 70, 80, 90, 100, 200, 300, 400, 500, 600, 700, 800, 900, 1000, 1500, 2000, 3000, 4000, 5000, 6000, 7000, 8000, 9000, 10,000, 11,000, 12,000, 13,000, 14,000, 15,000, 16,000, 17,000, 18,000, 19,000, 20,000, 30,000, 40,000, 50,000, 60,000, 70,000, 80,000, 90,000, 100,000, 200,000, 300,000, 400,000, 500,000, 600,000, 700,000, 800,000, 900,000, 1,000,000, 50,000,000, 100,000,000 different primers. For example, each primer in a plurality of primers can comprise a different target or template specific region or sequence.

### SEQUENCING

After performing one or more of the methods or method steps described herein, a library of polynucleotides generated can be sequenced.

Sequencing can be performed by any sequencing method known in the art. In some embodiments, sequencing can be performed in high throughput. Suitable next generation sequencing technologies include the 454 Life Sciences platform (Roche, Branford, CT) (Margulies et al., Nature, 437, 376-380 (2005)); Illumina's Genome Analyzer, GoldenGate Methylation Assay, or Infinium Methylation Assays, i.e., Infinium HumanMethylation 27K BeadArray or VeraCode GoldenGate methylation array (Illumina, San Diego, CA; Bibkova et al., Genome Res. 16, 383-393 (2006); and U.S. Patent Nos. 6,306,597, 7,598,035, 7,232,656), or DNA Sequencing by Ligation, SOLiD System (Applied Biosystems/Life Technologies; U.S. Patent Nos. 6,797,470, 7,083,917, 7,166,434, 7,320,865, 7,332,285, 7,364,858, and 7,429,453); or the Helicos True Single Molecule DNA sequencing technology (Harris et al., Science, 320, 106-109 (2008); and U.S. Patent Nos. 7,037,687, 7,645,596, 7,169,560, and7,769,400), the single molecule, real-time (SMRTTm) technology of Pacific Biosciences, and sequencing (Soni et al., Clin. Chem. 53, 1996-2001 (2007)). These systems allow multiplexed parallel sequencing of many polynucleotides isolated from a sample (Dear, Brief Funct. Genomic Proteomic, 1(4), 397-416 (2003) and McCaughan et al., J. Pathol., 220, 297-306 (2010)). In some embodiments, polynucleotides are sequenced by sequencing by ligation of dye-modified probes, pyrosequencing, or single-molecule sequencing. Determining the sequence of a polynucleotide may be performed by sequencing methods such as HelioscopeTM single molecule sequencing, Nanopore DNA sequencing, Lynx Therapeutics' Massively Parallel Signature Sequencing (MPSS), 454 pyrosequencing, Single Molecule real time (RNAP) sequencing, Illumina (Solexa) sequencing, SOLiD sequencing, Ion TorrentTM, Ion semiconductor sequencing, Single Molecule SMRT(TM) sequencing, Polony sequencing, DNA nanoball sequencing, and VisiGen Biotechnologies approach. Alternatively, determining the sequence of polynucleotides may use sequencing platforms, including, but not limited to, Genome Analyzer IIx, HiSeq, and MiSeq offered by Illumina, Single Molecule Real Time (SMRT^{™}) technology, such as the PacBio RS system offered by Pacific Biosciences (California) and the Solexa Sequencer, True Single Molecule Sequencing (tSMS^{™}) technology such as the HeliScope^{™} Sequencer offered by Helicos Inc. (Cambridge, MA). Sequencing can comprise MiSeq sequencing. Sequencing can comprise HiSeq sequencing. In some embodiments, determining the sequence of a polynucleotide comprises paired-end sequencing, nanopore sequencing, high-throughput sequencing, shotgun sequencing, dye-terminator sequencing, multiple-primer DNA sequencing, primer walking, Sanger dideoxy sequencing, Maxim-Gilbert sequencing, pyrosequencing, true single molecule sequencing, or any combination thereof. Alternatively, the sequence of a polynucleotide can be determined by electron microscopy or a chemicalsensitive field effect transistor (chemFET) array.

A method can further comprise sequencing one or more polynucleotides in the library. A method can further comprise aligning one or more polynucleotide sequences, sequence reads, amplicon sequences, or amplicon set sequences in the library to each other.

As used herein, aligning comprises comparing a test sequence, such as a sequence read, to one or more other test sequences, reference sequences, or a combination thereof. In some embodiments, aligning can be used to determine a consensus sequence from a plurality of sequences or aligned sequences. In some embodiments, aligning comprises determining a consensus sequence from a plurality of sequences that each has an identical molecular barcode or vessel barcode. In some embodiments, the length of a sequence aligned for comparison purposes is at least 30%, at least 40%, at least 50%, at least 60%, at least 70%, at least 80%, at least 90%, or at least 95%, of the length of a reference sequence. The actual comparison of the two or more sequences can be accomplished by well-known methods, for example, using a mathematical algorithm. A non-limiting example of such a mathematical algorithm is described in Karlin, S. and Altschul, S., Proc. Natl. Acad. Sci. USA, 90- 5873-5877 (1993). Such an algorithm is incorporated into the NBLAST and XBLAST programs (version 2.0), as described in Altschul, S. et al., Nucleic Acids Res., 25:3389-3402 (1997). When utilizing BLAST and Gapped BLAST programs, any relevant parameters of the respective programs (e.g., NBLAST) can be used. For example, parameters for sequence comparison can be set at score= 100, word length= 12, or can be varied (e.g., W=5 or W=20). Other examples include the algorithm of Myers and Miller, CABIOS (1989), ADVANCE, ADAM, BLAT, and FASTA. In some embodiments, the percent identity between two amino acid sequences can be accomplished using, for example, the GAP program in the GCG software package (Accelrys, Cambridge, UK).

Sequencing can comprise sequencing at least about 10, 20, 30, 40, 50, 60, 70, 80, 90, 100 or more nucleotides or base pairs of the polynucleotides. In some embodiments, sequencing comprises sequencing at least about 200, 300, 400, 500, 600, 700, 800, 900, 1000, or more nucleotides or base pairs of the polynucleotides. In other instances, sequencing comprises sequencing at least about 1500, 2000, 3000, 4000, 5000, 6000, 7000, 8000, 9000, 10,000, or more nucleotides or base pairs of the polynucleotides.

Sequencing can comprise at least about 200, 300, 400, 500, 600, 700, 800, 900, 1000 or more sequencing reads per run. As used herein, a sequence read comprises a sequence of nucleotides determined from a sequence or stream of data generated by a sequencing technique. In some embodiments, sequencing comprises sequencing at least about 1500, 2000, 3000, 4000, 5000, 6000, 7000, 8000, 9000, 10,000, or more sequencing reads per run. Sequencing can comprise more than, less than, or equal to about 1,000,000,000 sequencing reads per run. Sequencing can comprise more than, less than, or equal to about 200,000,000 reads per run.

In some embodiments, the number of sequence reads used to determine a consensus sequence is from about 2-1000 sequence reads. For example, the number of sequence reads used to determine a consensus sequence can be from about 2-900, 2-800, 2-700, 2-600, 2-500, 2-400, 2-300, 2-200, 2-100, 25-900, 25-800, 25-700, 25-600, 25-500, 25-400, 25-300, 25-200, 25-100, 100-1000, 100-900, 100-800, 100-700, 100-600, 100-500, 100-400, 100-300, 100-200, 200-1000, 200-900, 200-800, 200-700, 200-600, 200-500, 200-400, 200-300, 300-1000, 300-900, 300-800, 300-700, 300-600, 300-500, 300-400, 400-1000, 400-900, 400-800, 400-700, 400-600, 400-500, 500-1000, 500-900, 500-800, 500-700, 500-600, 600-1000, 600-900, 600-800, 600-700, 700-1000, 700-900, 700-800, 800-1000, 800-900, or 900-1000 sequence reads. In some embodiments, the number of sequence reads used to determine a consensus sequence is at least about 1000, 1500, 2000, 3000, 4000, 5000, 6000, 7000, 8000, 9000, 10,000, 11,000, 12,000, 13,000, 14,000, 15,000, 16,000, 17,000, 18,000, 19,000, 20,000, 25,000, 30,000,35,000, 40,000, 45,000, 50,000, 55,000, 60,000, 65,000, 70,000, 75,000, 80,000, 85,000, 90,000, 95000, 100,000, 150,000, 200,000, 250,000, 300,000, 350,000, 400,000, 450,000, 500,000, 550,000, 600,000, 650,000, 700,000, 750,000, 800,000, 850,000, 900,000, 950,000, 1,000,000, 50,000,000, or 100,000,000 reads. In some embodiments, the number of sequence reads used to determine a consensus sequence is at most about 1000, 1500, 2000, 3000, 4000, 5000, 6000, 7000, 8000, 9000, 10,000, 11,000, 12,000, 13,000, 14,000, 15,000, 16,000, 17,000, 18,000, 19,000, 20,000, 25,000, 30,000,35,000, 40,000, 45,000, 50,000, 55,000, 60,000, 65,000, 70,000, 75,000, 80,000, 85,000, 90,000, 95000, 100,000, 150,000, 200,000, 250,000, 300,000, 350,000, 400,000, 450,000, 500,000, 550,000, 600,000, 650,000, 700,000, 750,000, 800,000, 850,000, 900,000, 950,000, 1,000,000, 50,000,000, or 100,000,000 reads.

A method can comprise sequencing mis-reads. A method can comprise determining the number of mis-reads, such as for determining a reaction condition or designing primer sequences. Comparing the number of mis-reads generated under one or more first conditions or sets of conditions can be used to determine a preferred condition or condition set. For example, a first method can be carried out at a high salt concentration during a PCR reaction, and a second method can be carried out at a low salt concentration during a PCR reaction, wherein the first and second method are carried out substantially the same aside from the salt concentration difference. If the first method results in a higher number of mis-reads, such as a higher number of mis-reads for a particular target polynucleotide sequence or primer, a lower salt reaction condition can be determined to be preferred for that particular target polynucleotide sequence or primer.

### HITS

In one aspect, provided herein, but not part of the invention, is a kit comprising a plurality of template barcoded polynucleotides comprising an exclusionary sequence; and a plurality of primers comprising a first extension primer comprising an activating sequence with complementarity to an exclusionary sequence, and a second extension primer complementary to the activated barcoded polynucleotide.

In some embodiments, the kit further comprises a first amplification primer with complementarity to the exclusion sequence and a second amplification primer complementary to the activated barcoded polynucleotide.

In some embodiments, the kit further comprises an extension primer blocking oligonucleotide.

In some embodiments, the extension primer blocking oligonucleotide has a melting temperature to a template barcoded polynucleotide of the plurality of template barcoded polynucleotides that is higher than the melting temperature of the first extension primer to the template barcoded polynucleotide of the plurality of template barcoded polynucleotides.

In some embodiments, the extension primer blocking oligonucleotide has a melting temperature to a template barcoded polynucleotide of the plurality of template barcoded polynucleotides that is at least 1 °C, 2 °C, 3 °C, 4 °C, 5 °C, 6 °C, 7 °C, 8 °C, 9 °C, 10 °C, 11 °C, 12 °C, 13 °C, 14 °C, 15 °C, 16 °C, 17 °C, 18 °C, 19 °C, 20 °C, 21 °C, 22 °C, 23 °C, 24 °C, 25 °C, 26 °C, 27 °C, 28 °C, 29 °C, or 30 °C higher than the melting temperature of the first extension primer to the template barcoded polynucleotide of the plurality of template barcoded polynucleotides.

In some embodiments, the extension primer blocking oligonucleotide has a melting temperature to the plurality of template barcoded polynucleotides that is higher than the melting temperature of the first extension primer to the plurality of template barcoded polynucleotides.

In some embodiments, the extension primer blocking oligonucleotide is longer than the first extension primer.

In some embodiments, the extension primer blocking oligonucleotide hybridizes to a region of the plurality of template barcoded polynucleotides comprising the exclusionary sequence.

In some embodiments, the region of the plurality of template barcoded polynucleotides comprising the exclusionary sequence to which the extension primer blocking oligonucleotide hybridizes is longer than the exclusionary sequence.

### COMPOSITIONS

In one aspect, provided herein, but not part of the invention, is a composition comprising a plurality of vessels, each vessel of the plurality comprising a plurality of template barcoded polynucleotide molecules and a target polynucleotide comprising a barcode amplified from a single template barcoded polynucleotide molecule of the plurality of template barcoded polynucleotide molecules.

In one aspect, provided herein is a composition, the composition comprising a plurality of vessels, each vessel of the plurality comprising a plurality of template barcoded polynucleotides each with a different barcode, and a target polynucleotide comprising a unique barcode amplified from a template barcoded polynucleotide of the plurality of template barcoded polynucleotides.

In one aspect, provided herein is a composition comprising a plurality of vessels, each vessel of the plurality comprising a plurality of template barcoded polynucleotides, a first primer with an affinity to each template barcoded polynucleotide that is about the same, a second primer with an affinity to an extension product of the first primer that is as least 5-fold more than the affinity of the first primer to each of the template barcoded polynucleotides, and a target polynucleotide comprising a unique barcode amplified from a template barcoded polynucleotide a of the plurality of template barcoded polynucleotides.

In some embodiments, the plurality of vessels comprises at least 50 vessels. In some embodiments, each vessel of the plurality comprises an extension primer blocking oligonucleotide. In some embodiments, each vessel of the plurality comprises a polymerase. In some embodiments, each vessel of the plurality comprises a reverse transcriptase.

While some embodiments described herein have been shown and described herein, it will be obvious to those skilled in the art that such embodiments are provided by way of example only. Numerous variations, changes, and substitutions will now occur to those skilled in the art without departing from the disclosure provided herein. It should be understood that various alternatives to the embodiments described herein can be employed in practicing the methods described herein.

### EXAMPLES

### Example 1

Real-time PCR reactions and 3-color Taqman assays were performed within droplets using activated droplet exclusion PCR methods. A comparison of the results to simulations demonstrated that the methods work very closely to the level predicted by theory, and were effective to overcome Poisson distribution limitations of other droplet barcoding methods. These results also indicated the activated droplet exclusion PCR methods described herein would be applicable to and would be useful for methods in which single DNA templates are desired to be amplified within each of a number of isolated reaction volumes.

### Example 2 - Method for preventing extension of an extension primer during reverse transcription (RT) before PCR for RT-PCR applications.

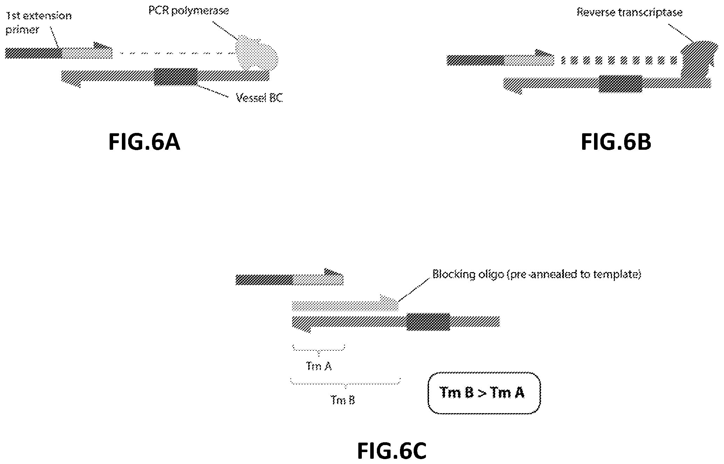

Reverse transcription and PCR reactions were performed within droplets using activated droplet exclusion PCR methods. Reverse transcription and PCR reactions were performed within droplets containing a polymerase, within droplets containing a polymerase and a reverse transcriptase, and within droplets containing a polymerase, a reverse transcriptase, and an extension primer blocking oligonucleotide (**FIG. 6**). The extension primer blocking oligonucleotide was designed to have a higher T_{M} than the T_{M} of the extension primer.

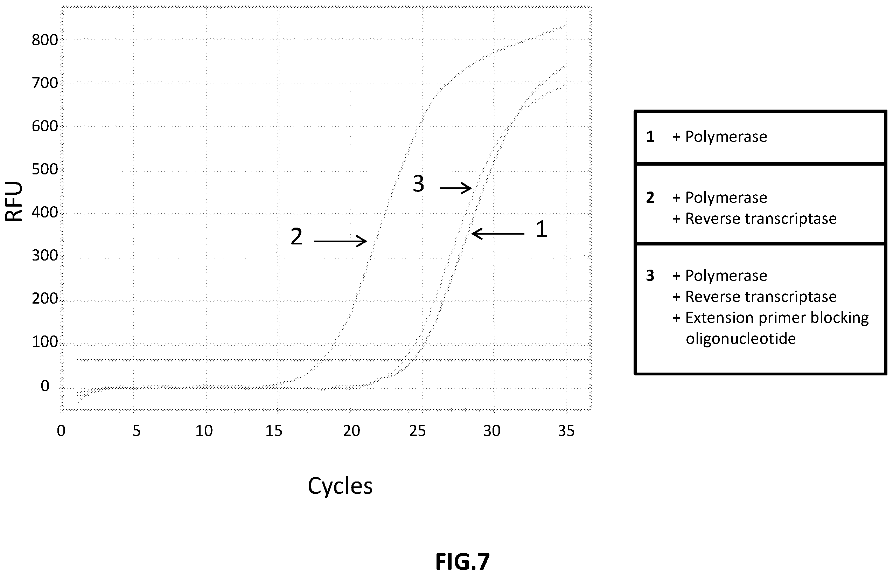

Real-time PCR was carried out to amplify 1× 10⁷ templates in the presence of a low-efficiency extension primer and (1) a PCR polymerase, (2) a PCR polymerase and a reverse transcriptase, or (3) a PCR polymerase, a reverse transcriptase, and an extension primer blocking oligonucleotide pre-annealed to the templates. A comparison of the results (**FIG. 7**) suggests that within droplets containing a polymerase and a reverse transcriptase without an extension primer blocking oligonucleotide, the extension primer annealed to a template barcoded polynucleotide during reverse transcription, and was extended before PCR. However, within droplets containing a polymerase, a reverse transcriptase, and an extension primer blocking oligonucleotide, FIG. 7 suggests that annealing of the extension primer blocking oligonucleotide to the template barcoded polynucleotide prevented access of the extension primer to the template during reverse transcription, and prevented extension of the extension primer before PCR.

## Claims

1. A method of producing amplicons derived from a single template barcoded polynucleotide molecule that comprises an exclusion sequence and a barcode in a plurality of vessels comprising a plurality of template barcoded polynucleotide molecules the method comprising:
(a) performing a first reaction, whereby a first extension primer comprising an activating sequence anneals to the exclusion sequence in the single template barcoded polynucleotide molecule and is extended, forming an activated barcoded polynucleotide, said activating sequence in the first extension primer comprising at least 2, 3, 4, 5, 6, 7, 8, 9, or 10 mismatches to the exclusion sequence in the template barcoded polynucleotide;
(b) performing a second reaction whereby a second extension primer anneals to the activated barcoded polynucleotide, forming a polynucleotide complementary to the activated barcoded polynucleotide and lacking the exclusion sequence ("nonexclusionary template barcoded polynucleotide") and
(c) amplifying the activated barcoded polynucleotide and the nonexclusionary template barcoded polynucleotide using a first amplification primer comprising the activating sequence of the first extension primer and a second amplification primer complementary to a sequence of the activated barcoded polynucleotide, forming amplicons comprising a barcode amplified from the single template barcoded polynucleotide molecule;
wherein the plurality of vessels comprises at least 50 vessels.

2. The method of claim 1, wherein, in 80-100% of the vessels of the plurality of vessels, 80-100% of the amplicons formed by the method comprise a barcode amplified from the single template barcoded polynucleotide molecule.

3. The method of claim 1 or claim 2, wherein the plurality of vessels comprises from 1-1000 template barcoded polynucleotide molecules per vessel, optionally from 2-1000 template barcoded polynucleotides per vessel, optionally from 1-6 template barcoded polynucleotide molecules per vessel.

4. The method of any one of claims 1-3, wherein, in the plurality of vessels,
(a) the extension efficiency of the first reaction is once per two PCR cycles and wherein the extension efficiency of the second reaction is at least about 2-fold higher than the extension efficiency of the first reaction; or
(b) the extension efficiency of the first reaction is about once per 3, 4, 5, 6, 7, 8, 9, 10, 11, 12, 13, 14, or 15 PCR cycles;
and optionally wherein:
(i) the extension efficiency by the first extension primer in the first reaction is at least 5-fold less than the extension efficiency in the second reaction; or
(ii) the extension efficiency of the first reaction is at least about 10-fold, 25-fold, 50-fold, 100-fold, or 1000-fold lower than the extension efficiency of the second reaction and/or an extension reaction of the amplifying; or
(iii) the extension efficiency of the first reaction is from about 0.0005-20%, from about 0.005%-5%, or from about 0.05%-0.5%; wherein the second extension primer comprises 80-100% complementarity to a region of the activated barcoded polynucleotide that is more than 8 bases in length.

5. The method of any one of claims 1-4, wherein the first extension primer has a melting temperature to the single template barcoded polynucleotide molecule that is lower than the melting temperature of the second extension primer to an extension product of the first extension primer, optionally wherein the first extension primer has a melting temperature to the single template barcoded polynucleotide that is lower than an annealing temperature of a PCR cycle by at least 1 °C, 2 °C, 3 °C, 4 °C, 5 °C, 6 °C, 7 °C, 8 °C, 9 °C, 10 °C, 11 °C, 12 °C, 13 °C, 14 °C, or 15 °C.

6. The method of any one of claims 1-5, wherein the target polynucleotide is RNA and the method further comprises performing a reverse transcription reaction.

7. The method of any one of claims 1-6, wherein the method further comprises sequencing one or more polynucleotides from the plurality of vessels.

8. The method of any one of claims 1-7, wherein the first reaction and the second reaction occur during different PCR cycles.

9. The method of any one of claims 1-8, wherein the vessel is a well, an emulsion, or a droplet.

10. The method of any one of claims 1-9, wherein the amplifying comprises performing a third reaction comprising extending the first extension primer annealed to the nonexclusionary template barcoded polynucleotide.

11. The method of any one of claims 1-10, wherein the single template barcoded polynucleotide molecule and/or first extension primer is distributed within the plurality of vessels in a Poisson distribution.

12. The method of any one of claims 1-11, wherein, in the plurality of vessels, the activating sequence of the first extension primer has less than 95%, 90%, 85%, 80%, 75%, 70%, 65%, 60%, 55%, or 50% sequence complementarity to the exclusion sequence, optionally wherein the activating sequence comprises at least 2, 3, 4, 5, 6, 7, 8, 9, or 10 mismatches to the exclusion sequence.

13. The method of any one of claims 1-12, wherein at least 70%, 80%, 90%, 95%, 98%, or 99%, or 100% of the plurality of vessels comprises a cell, optionally a single cell.

14. The method of claim 6, wherein the plurality of vessels further comprises an extension primer blocking oligonucleotide that anneals to a region of the single template barcoded polynucleotide molecule,
optionally wherein the first extension primer or an amplification primer anneals to a first region of the single template barcoded polynucleotide molecule and the extension primer blocking oligonucleotide anneals to a second region thereof, wherein the first region and second region overlap, and further optionally wherein the first region is shorter than the second region.

15. The method of claim 14, wherein the extension primer blocking oligonucleotide has a melting temperature to at least one of the single template barcoded polynucleotide molecule that is higher than the melting temperature of an extension primer or amplification primer to said single template barcoded polynucleotide molecule.

## Patentansprüche

1. Ein Verfahren zur Herstellung von Amplicons, die aus einem strichcodierten Einzelmatrizen-Polynukleotidmolekül abgeleitet sind, das eine Ausschlusssequenz und einen Strichcode in einer Vielzahl von Gefäßen umfasst, die eine Vielzahl von strichcodierten Matrizen-Polynukleotidmolekülen umfassen, wobei das Verfahren Folgendes umfasst:
(a) Ausführen einer ersten Reaktion, wodurch ein erster Extension-Primer, der eine Aktivierungssequenz umfasst, mit der Ausschlusssequenz in dem strichcodierten Einzelmatrizen-Polynukleotidmolekül hybridisiert und durch Formen eines aktivierten strichcodierten Polynukleotids erweitert wird, diese Aktivierungssequenz in dem ersten Extension-Primer umfasst mindestens 2, 3, 4, 5, 6, 7, 8, 9 oder 10 Fehlanpassungen an die Ausschlusssequenz in dem strichcodierten Matrizen-Polynukleotid;
(b) Ausführen einer zweiten Reaktion, wodurch ein zweiter Extension-Primer mit dem aktivierten strichcodierten Polynukleotid hybridisiert und eine Polynukleotid-Ergänzung zu dem aktivierten strichcodierten Polynukleotid bildet und keine Ausschusssequenz aufweist ("nicht ausschließendes strichcodiertes Matrizen-Polynukleotid") und
(c) Verstärken des aktivierten strichcodierten Polynukleotids und des nicht ausschließenden strichcodierten Matrizen-Polynukleotids unter Verwendung eines ersten Amplifikationsprimers, der die Aktivierungssequenz des ersten Extension-Primers umfasst, und eines zweiten Amplifikationsprimers, als Ergänzung zu einer Sequenz des aktivierten strichcodierten Polynukleotids, der Amplicons bildet, die einen Strichcode umfassen, der mit dem strichcodierten Einzelmatrizen-Polynukleotidmolekül verstärkt wird;
wobei die Vielzahl der Gefäße mindestens 50 Gefäße umfasst.

2. Verfahren nach Anspruch 1, wobei in 80 - 100 % der Gefäße der Vielzahl von Gefäßen, 80 - 100 % der Amplicons, die von dem Verfahren gebildet werden, einen Strichcode umfassen, der von dem strichcodierten Einzelmatrizen-Polynukleotidmolekül verstärkt wird.

3. Verfahren nach Anspruch 1 oder Anspruch 2, wobei die Vielzahl der Gefäße von 1 - 1000 strichcodierten Matrizen-Polynukleotidmolekülen pro Gefäß, optional von 2 - 1000 strichcodierten Matrizen-Polynukleotidmolekülen pro Gefäß, optional von 1 - 6 strichcodierten Matrize-Polynukleotidmolekülen pro Gefäß umfasst.

4. Verfahren nach einem der Ansprüche 1-3, wobei in der Vielzahl der Gefäße,
(a) die Erweiterungseffizienz der ersten Reaktion einmal pro zwei PCR-Zyklen beträgt und wobei die Erweiterungseffizienz der zweiten Reaktion mindestens ungefähr 2-fach höher ist als die Erweiterungseffizienz der ersten Reaktion; oder
(b) die Erweiterungseffizienz der ersten Reaktion ungefähr einmal pro 3, 4, 5, 6, 7, 8, 9, 10, 11, 12, 13, 14 oder 15 PCR-Zyklen beträgt;
und optional wobei:
(i) die Erweiterungseffizienz durch den ersten Extension-Primer in der ersten Reaktion mindestens 5-fach geringer ist als die Erweiterungseffizienz in der zweiten Reaktion; oder
(ii) die Erweiterungseffizienz der ersten Reaktion mindestens ungefähr 10-fach, 25-fach, 50-fach, 100-fach oder 1000-fach geringer ist als die Erweiterungseffizienz der zweiten Reaktion und/oder eine Erweiterungsreaktion der Verstärkung; oder
(iii) die Erweiterungseffizienz der ersten Reaktion von ungefähr 0,0005 - 20 %, von ungefähr 0,005 % - 5 % oder von ungefähr 0,05 % - 0,5 % beträgt; wobei der zweite Extension-Primer 80 - 100 % Komplementarität zu einer Region des aktivierten strichcodierten Polynukleotids umfasst, die mehr als 8 Basen in der Länge aufweist.

5. Verfahren nach einem der Ansprüche 1 - 4, wobei der erste Extension-Primer eine Schmelztemperatur an dem strichcodierten Einzelmatrizen-Polynukleotidmolekül aufweist, die geringer ist als die Schmelztemperatur des zweiten Extension-Primers an einem Erweiterungsprodukt des ersten Extension-Primers, optional wobei der erste Extension-Primer eine Schmelztemperatur an dem strichcodierten Einzelmatrizen-Polynukleotid aufweist, die geringer ist als eine Annealing-Temperatur eines PCR-Zyklus um mindestens 1 °C, 2 °C, 3 °C, 4 °C, 5 °C, 6 °C, 7 °C, 8 °C, 9 °C, 10 °C, 11 °C, 12 °C, 13 °C, 14 °C oder 15 °C.

6. Verfahren nach einem der Ansprüche 1 - 5, wobei das Ziel-Polynukleotid RNA ist und das Verfahren ferner das Ausführen einer Umkehrtranskriptions-Reaktion umfasst.

7. Verfahren nach einem der Ansprüche 1 - 6, wobei das Verfahren ferner das Sequenzieren eines oder mehrerer Polynukleotide aus der Vielzahl der Gefäße umfasst.

8. Verfahren nach einem der Ansprüche 1 - 7, wobei die erste Reaktion und die zweite Reaktion während verschiedener PCR-Zyklen auftreten.

9. Verfahren nach einem der Ansprüche 1 - 8, wobei das Gefäß ein Well, eine Emulsion oder ein Tröpfchen ist.

10. Verfahren nach einem der Ansprüche 1 - 9, wobei das Amplifizieren das Ausführen einer dritten Reaktion umfasst, die das Erweitern des ersten Extension-Primers umfasst, der mit dem nicht ausschließenden strichcodierten Matrizen-Polynukleotid hybridisiert ist.

11. Verfahren nach einem der Ansprüche 1 - 10, wobei das strichcodierte Einzelmatrizen-Polynukleotidmolekül und/oder der erste Extension-Primer innerhalb der Vielzahl von Gefäßen in einer Poisson-Verteilung verteilt ist.

12. Verfahren nach einem der Ansprüche 1 - 11, wobei in der Vielzahl von Gefäßen die Aktivierungssequenz des ersten Extension-Primers weniger als 95 %, 90 %, 85 %, 80 %, 75 %, 70 %, 65 %, 60 %, 55 % oder 50 % Sequenz-Komplementarität zu der Ausschlusssequenz hat, optional wobei die Aktivierungssequenz mindestens 2, 3, 4, 5, 6, 7, 8, 9 oder 10 Fehlanpassungen an die Ausschlusssequenz hat.

13. Verfahren nach einem der Ansprüche 1 - 12, wobei mindestens 70 %, 80 %, 90 %, 95 %, 98 % oder 99 % oder 100 % der Vielzahl von Gefäßen eine Zelle, optional eine Einzelzelle umfasst.

14. Verfahren nach Anspruch 6, wobei die Vielzahl der Gefäße ferner einen Extension-Primer umfasst, der das Oligonukleotid blockiert, das eine Region des strichcodierten Einzelmatrizen-Polynukleotidmoleküls hybridisiert,
optional wobei der erste Extension-Primer oder ein Amplifikationsprimer mit einer ersten Region des strichcodierten Einzelmatrizen-Polynukleotidmoleküls hybridisiert, und der Extension-Primer, der Oligonukleotid blockiert, mit einer zweiten Region davon hybridisiert, wobei die erste Region und die zweite Region überlappen, und ferner optional wobei die erste Region kürzer ist als die zweite Region.

15. Verfahren nach Anspruch 14, wobei der Extension-Primer, der Oligonukleotid blockiert, eine Schmelztemperatur für mindestens eines der strichcodierten Einzelmatrizen-Polynukleotidmoleküle aufweist, die höher ist als die Schmelztemperatur eines Extension-Primers oder Amplifikationsprimers für das strichcodierte Einzelmatrizen-Polynukleotidmolekül.

## Revendications

1. Procédé de fabrication d'amplicons dérivés d'une molécule de polynucléotide codé à barres matriciel unique qui comprend une séquence d'exclusion et un code à barres dans une pluralité de récipients comprenant une pluralité de molécules de polynucléotide codé à barres matriciel, le procédé comprenant :
(a) la réalisation d'une première réaction grâce à laquelle une première amorce d'extension comprenant une séquence d'activation s'hybride à la séquence d'exclusion dans la molécule de polynucléotide codé à barres matriciel unique et est étendue, formant un polynucléotide codé à barres activé, ladite séquence d'activation dans la première amorce d'extension comprenant au moins 2, 3, 4, 5, 6, 7, 8, 9 ou 10 mésappariements par rapport à la séquence d'exclusion dans le polynucléotide codé à barres matriciel ;
(b) la réalisation d'une deuxième réaction grâce à laquelle une seconde amorce d'extension s'hybride au polynucléotide codé à barres activé, formant un polynucléotide complémentaire au polynucléotide codé à barres activé et dépourvu de la séquence d'exclusion (« polynucléotide codé à barres matriciel non exclusif »), et
(c) l'amplification du polynucléotide codé à barres activé et le polynucléotide codé à barres matriciel non exclusif à l'aide d'une première amorce d'amplification comprenant la séquence d'activation de la première amorce d'extension et d'une seconde amorce d'amplification complémentaire à une séquence du polynucléotide codé à barres activé, formant des amplicons comprenant un code à barres amplifié à partir de la molécule de polynucléotide codé à barres matriciel unique ;
dans lequel la pluralité de récipients comprend au moins 50 récipients.

2. Procédé selon la revendication 1, dans lequel, dans 80 à 100 % des récipients de la pluralité de récipients, 80 à 100 % des amplicons formés par le procédé comprennent un code à barres amplifié à partir de la molécule de polynucléotide codé à barres matriciel unique.

3. Procédé selon la revendication 1 ou la revendication 2, dans lequel la pluralité de récipients comprend de 1 à 1 000 molécules de polynucléotide codé à barres matriciel par récipient, éventuellement de 2 à 1 000 polynucléotides codé à barres par récipient, éventuellement de 1 à 6 molécules de polynucléotide codé à barres matriciel par récipient.

4. Procédé selon l'une quelconque des revendications 1 à 3, dans lequel dans la pluralité de récipients,
(a) l'efficacité d'extension de la première réaction est une fois par deux cycles de PCR (réaction en chaîne de polymérase) et dans lequel l'efficacité d'extension de la deuxième réaction est au moins environ deux fois plus élevée que l'efficacité d'extension de la première réaction ; ou
(b) l'efficacité d'extension de la première réaction est d'environ une fois par 3, 4, 5, 6, 7, 8, 9, 10, 11, 12, 13, 14 ou 15 cycles de PCR ;
et éventuellement, dans lequel :
(i) l'efficacité d'extension par la première amorce d'extension dans la première réaction est au moins 5 fois inférieure à l'efficacité d'extension dans la deuxième réaction ; ou
(ii) l'efficacité d'extension de la première réaction est au moins environ 10 fois, 25 fois, 50 fois, 100 fois ou 1000 fois inférieure à l'efficacité d'extension de la deuxième réaction et/ou d'une réaction d'extension de l'amplification ; ou
(iii) l'efficacité d'extension de la première réaction est d'environ 0,0005 à 20 %, d'environ 0,005 % à 5 %, ou d'environ 0,05 % à 0,5 % ; dans lequel la seconde amorce d'extension présente une complémentarité de 80-100 % avec une région du polynucléotide codé à barres activé d'une longueur de plus de 8 bases.

5. Procédé selon l'une des revendications 1 à 4, dans lequel la première amorce d'extension présente une température de fusion avec la molécule de polynucléotide codé à barres matriciel unique qui est inférieure à la température de fusion de la seconde amorce d'extension avec un produit d'extension de la première amorce d'extension, éventuellement, dans lequel la première amorce d'extension présente une température de fusion avec le polynucléotide codé à barres à matrice unique qui est inférieure à une température d'hybridation d'un cycle de PCR d'au moins 1 °C, 2 °C, 3 °C, 4 °C, 5 °C, 6 °C, 7 °C, 8 °C, 9 °C, 10 °C, 11 °C, 12 °C, 13 °C, 14 °C ou 15 °C.

6. Procédé selon l'une des revendications 1 à 5, dans lequel le polynucléotide cible est l'ARN et le procédé comprend en outre la réalisation d'une réaction de transcription inverse.

7. Procédé selon l'une des revendications 1 à 6, dans lequel le procédé comprend en outre le séquençage d'un ou plusieurs polynucléotides provenant de la pluralité de récipients.

8. Procédé selon l'une des revendications 1 à 7, dans lequel la première réaction et la deuxième réaction se produisent au cours de cycles de PCR différents.

9. Procédé selon l'une quelconque des revendications 1 à 8, dans lequel le récipient est un puits, une émulsion ou une goutte.

10. Procédé selon l'une des revendications 1 à 9, dans lequel l'amplification comprend la réalisation d'une troisième réaction comprenant l'extension de la première amorce d'extension hybridée au polynucléotide codé à barres matriciel non exclusif.

11. Procédé selon l'une des revendications 1 à 10, dans lequel la molécule de polynucléotide codé à barres matriciel unique et/ou la première amorce d'extension sont distribuées dans la pluralité de récipients selon une distribution de Poisson.

12. Procédé selon l'une des revendications 1 à 11, dans lequel, dans la pluralité de récipients, la séquence d'activation de la première amorce d'extension présente une complémentarité de séquence inférieure à 95 %, 90 %, 85 %, 80 %, 75 %, 70 %, 65 %, 60 %, 55 % ou 50 % par rapport à la séquence d'exclusion, la séquence d'activation comprenant éventuellement au moins 2, 3, 4, 5, 6, 7, 8, 9 ou 10 mésappariements par rapport à la séquence d'exclusion.

13. Procédé selon l'une des revendications 1 à 12, dans lequel au moins 70 %, 80 %, 90 %, 95 %, 98 %, 99 % ou 100 % de la pluralité de récipients comprend une cellule, éventuellement une cellule unique.

14. Procédé selon la revendication 6, dans lequel la pluralité de récipients comprend en outre un oligonucléotide de blocage de l'amorce d'extension qui s'hybride à une région de la molécule de polynucléotide codé à barres matriciel unique,
éventuellement, dans lequel la première amorce d'extension ou une amorce d'amplification s'hybride à une première région de la molécule de polynucléotide codé à barres matriciel unique et l'oligonucléotide de blocage de l'amorce d'extension s'hybride à une deuxième région de celle-ci, dans lequel la première région et la deuxième région se chevauche et en outre, éventuellement, dans lequel la première région est plus courte que la seconde région.

15. Procédé selon la revendication 14, dans lequel l'oligonucléotide de blocage de l'amorce d'extension a une température de fusion avec au moins une molécule de polynucléotide codé à barres matriciel unique qui est supérieure à la température de fusion d'une amorce d'extension ou d'une amorce d'amplification avec ladite molécule de polynucléotide codé à barres matriciel unique.
